# EUROPEAN PATENT APPLICATION

(11) **EP 1 865 063 A1**
(43) Date of publication of application: **12.12.2007**
(21) Application number: 06729595.6
(22) Date of filing: 15.03.2006
(51) Int. Cl.: C12N 15/60, C12N 1/15, C12N 1/19, C12N 1/21, C12N 5/10, C12N 9/88, C12Q 1/527

(54) **GLUTAMATE DECARBOXYLASE MUTANT**

(30) Priority: 15.03.2005 JP 2005074056
(71) Applicant: Tokyo University of Science, Educational Foundation, Tokyo 162-8601 (JP)
(72) Inventor: YOSHIDA, Michiteru, home, Shinjuku-ku, Tokyo, 1628601 (JP); FUKASAWA, Kenji, home, Shinjuku-ku, Tokyo, 1628601 (JP); UEDA, Tetsuya, Chiba 2850861 (JP); TANNO, Kazunobu, Ibaraki 3160036 (JP)
(74) Representative: Denison, Christopher Marcus
(86) International application number: PCT/JP2006/305628
(87) International publication number: WO 2006/098485

(57) **Abstract**

Thermally stable GAD65 mutants are provided, which are useful for early diagnosis of insulin-dependent diabetes mellitus and retain antigenicity to pancreatic islet-β-cell-specific autoantibodies. Specifically, error-prone PCR is performed using a GAD65 gene as a template. A host microorganism is transformed with the thus obtained random gene library. Screening is performed using as an indicator the presence or the absence of antigenicity of the expressed protein to a pancreatic islet-β-cell-specific autoantibody after heat treatment. Thus, a GAD65 mutant having thermostability and antigenicity can be obtained.

SEQUENCE LISTING

<110> Tokyo University of Sciense

<120> Glutamic acid decarboxylase 65 mutants

<130> PH-2771-PCT

<150> JP 2005-074056
<151> 2005-03-15

<160> 32

<170> PatentIn version 3.1

<210> 1
<211> 1776
<212> DNA
<213> human GAD65

<220>
<221> CDS
<222> (1)..(1773)
<223>

<400> 1
atg gca tct ccg ggc tct ggc ttt tgg tct ttc ggg tcg gaa gat ggc 48
Met Ala Ser Pro Gly Ser Gly Phe Trp Ser Phe Gly Ser Glu Asp Gly
1               5                   10                  15

tct ggg gat tcc gag aat ccc ggc aca gcg cga gcc tgg tgc caa gtg 96
Ser Gly Asp Ser Glu Asn Pro Gly Thr Ala Arg Ala Trp Cys Gln Val
            20                  25                  30

gct cag aag ttc acg ggc ggc atc gga aac aaa ctg tgc gcc ctg ctc 144
Ala Gln Lys Phe Thr Gly Gly Ile Gly Asn Lys Leu Cys Ala Leu Leu
        35                  40                  45

tac gga gac gcc gag aag ccg gcg gag agc ggc ggg agc caa ccc ccg 192
Tyr Gly Asp Ala Glu Lys Pro Ala Glu Ser Gly Gly Ser Gln Pro Pro
    50                  55                  60

cgg gcc gcc gcc cgg aag gcc gcc tgc gcc tgc gac cag aag ccc tgc 240
Arg Ala Ala Ala Arg Lys Ala Ala Cys Ala Cys Asp Gln Lys Pro Cys
65                  70                  75                  80

agc tgc tcc aaa gtg gat gtc aac tac gcg ttt ctc cat gca aca gac 288
Ser Cys Ser Lys Val Asp Val Asn Tyr Ala Phe Leu His Ala Thr Asp
                85                  90                  95

ctg ctg ccg gcg tgt gat gga gaa agg ccc act ttg gcg ttt ctg caa 336
Leu Leu Pro Ala Cys Asp Gly Glu Arg Pro Thr Leu Ala Phe Leu Gln
            100                 105                 110

gat gtt atg aac att tta ctt cag tat gtg gtg aaa agt ttc gat aga 384
Asp Val Met Asn Ile Leu Leu Gln Tyr Val Val Lys Ser Phe Asp Arg
        115                 120                 125

tca acc aaa gtg att gat ttc cat tat cct aat gag ctt ctc caa gaa 432
Ser Thr Lys Val Ile Asp Phe His Tyr Pro Asn Glu Leu Leu Gln Glu
    130                 135                 140

tat aat tgg gaa ttg gca gac caa cca caa aat ttg gag gaa att ttg 480
Tyr Asn Trp Glu Leu Ala Asp Gln Pro Gln Asn Leu Glu Glu Ile Leu
145                 150                 155                 160

atg cat tgc caa aca act cta aaa tat gca att aaa aca ggg cat cct 528
Met His Cys Gln Thr Thr Leu Lys Tyr Ala Ile Lys Thr Gly His Pro
                165                 170                 175

aga tac ttc aat caa ctt tct act ggt ttg gat atg gtt gga tta gca 576
Arg Tyr Phe Asn Gln Leu Ser Thr Gly Leu Asp Met Val Gly Leu Ala
            180                 185                 190

gca gac tgg ctg aca tca aca gca aat act aac atg ttc acc tat gaa 624
Ala Asp Trp Leu Thr Ser Thr Ala Asn Thr Asn Met Phe Thr Tyr Glu
        195                 200                 205

att gct cca gta ttt gtg ctt ttg gaa tat gtc aca cta aag aaa atg 672
Ile Ala Pro Val Phe Val Leu Leu Glu Tyr Val Thr Leu Lys Lys Met
    210                 215                 220

aga gaa atc att ggc tgg cca ggg ggc tct ggc gat ggg ata ttt tct 720
Arg Glu Ile Ile Gly Trp Pro Gly Gly Ser Gly Asp Gly Ile Phe Ser
225                 230                 235                 240

ccc ggt ggc gcc ata tct aac atg tat gcc atg atg atc gca cgc ttt 768

Pro Gly Gly Ala Ile Ser Asn Met Tyr Ala Met Met Ile Ala Arg Phe
                245                 250                 255

aag atg ttc cca gaa gtc aag gag aaa gga atg gct gct ctt ccc agg 816
Lys Met Phe Pro Glu Val Lys Glu Lys Gly Met Ala Ala Leu Pro Arg
            260                 265                 270

ctc att gcc ttc acg tct gaa cat agt cat ttt tct ctc aag aag gga 864
Leu Ile Ala Phe Thr Ser Glu His Ser His Phe Ser Leu Lys Lys Gly
        275                 280                 285

gct gca gcc tta ggg att gga aca gac agc gtg att ctg att aaa tgt 912
Ala Ala Ala Leu Gly Ile Gly Thr Asp Ser Val Ile Leu Ile Lys Cys
    290                 295                 300

gat gag aga ggg aaa atg att cca tct gat ctt gaa aga agg att ctt 960
Asp Glu Arg Gly Lys Met Ile Pro Ser Asp Leu Glu Arg Arg Ile Leu
305                 310                 315                 320

gaa gcc aaa cag aaa ggg ttt gtt cct ttc ctc gtg agt gcc aca gct 1008
Glu Ala Lys Gln Lys Gly Phe Val Pro Phe Leu Val Ser Ala Thr Ala
                325                 330                 335

gga acc acc gtg tac gga gca ttt gac ccc ctc tta gct gtc gct gac 1056
Gly Thr Thr Val Tyr Gly Ala Phe Asp Pro Leu Leu Ala Val Ala Asp
            340                 345                 350

att tgc aaa aag tat aag atc tgg atg cat gtg gat gca gct tgg ggt 1104
Ile Cys Lys Lys Tyr Lys Ile Trp Met His Val Asp Ala Ala Trp Gly

355                 360                 365

ggg gga tta ctg atg tcc cga aaa cac aag tgg aaa ctg agt ggc gtg     1152
Gly Gly Leu Leu Met Ser Arg Lys His Lys Trp Lys Leu Ser Gly Val
    370                 375                 380

gag agg gcc aac tct gtg acg tgg aat cca cac aag atg atg gga gtc     1200
Glu Arg Ala Asn Ser Val Thr Trp Asn Pro His Lys Met Met Gly Val
385                 390                 395                 400

cct ttg cag tgc tct gct ctc ctg gtt aga gaa gag gga ttg atg cag     1248
Pro Leu Gln Cys Ser Ala Leu Leu Val Arg Glu Glu Gly Leu Met Gln
                405                 410                 415

aat tgc aac caa atg cat gcc tcc tac ctc ttt cag caa gat aaa cat     1296
Asn Cys Asn Gln Met His Ala Ser Tyr Leu Phe Gln Gln Asp Lys His
            420                 425                 430

tat gac ctg tcc tat gac act gga gac aag gcc tta cag tgc gga cgc     1344
Tyr Asp Leu Ser Tyr Asp Thr Gly Asp Lys Ala Leu Gln Cys Gly Arg
        435                 440                 445

cac gtt gat gtt ttt aaa cta tgg ctg atg tgg agg gca aag ggg act     1392
His Val Asp Val Phe Lys Leu Trp Leu Met Trp Arg Ala Lys Gly Thr
    450                 455                 460

acc ggg ttt gaa gcg cat gtt gat aaa tgt ttg gag ttg gca gag tat     1440
Thr Gly Phe Glu Ala His Val Asp Lys Cys Leu Glu Leu Ala Glu Tyr
465                 470                 475                 480

tta tac aac atc ata aaa aac cga gaa gga tat gag atg gtg ttt gat 1488
Leu Tyr Asn Ile Ile Lys Asn Arg Glu Gly Tyr Glu Met Val Phe Asp
                485                 490                 495

ggg aag cct cag cac aca aat gtc tgc ttc tgg tac att cct cca agc 1536
Gly Lys Pro Gln His Thr Asn Val Cys Phe Trp Tyr Ile Pro Pro Ser
            500                 505                 510

ttg cgt act ctg gaa gac aat gaa gag aga atg agt cgc ctc tcg aag 1584
Leu Arg Thr Leu Glu Asp Asn Glu Glu Arg Met Ser Arg Leu Ser Lys
        515                 520                 525

gtg gct cca gtg att aaa gcc aga atg atg gag tat gga acc aca atg 1632
Val Ala Pro Val Ile Lys Ala Arg Met Met Glu Tyr Gly Thr Thr Met
    530                 535                 540

gtc agc tac caa ccc ttg gga gac aag gtc aat ttc ttc cgc atg gtc 1680
Val Ser Tyr Gln Pro Leu Gly Asp Lys Val Asn Phe Phe Arg Met Val
545                 550                 555                 560

atc tca aac cca gcg gca act cac caa gac att gac ttc ctg att gaa 1728
Ile Ser Asn Pro Ala Ala Thr His Gln Asp Ile Asp Phe Leu Ile Glu
                565                 570                 575

gaa ata gaa cgc ctt gga caa gat tta cac cac cac cac cac cac taa 1776
Glu Ile Glu Arg Leu Gly Gln Asp Leu His His His His His His
            580                 585                 590

<210> 2

<211> 591

<212> PRT

<213> human GAD65

<400> 2

Met Ala Ser Pro Gly Ser Gly Phe Trp Ser Phe Gly Ser Glu Asp Gly
1               5                   10                  15

Ser Gly Asp Ser Glu Asn Pro Gly Thr Ala Arg Ala Trp Cys Gln Val
            20                  25                  30

Ala Gln Lys Phe Thr Gly Gly Ile Gly Asn Lys Leu Cys Ala Leu Leu
        35                  40                  45

Tyr Gly Asp Ala Glu Lys Pro Ala Glu Ser Gly Gly Ser Gln Pro Pro
    50                  55                  60

Arg Ala Ala Ala Arg Lys Ala Ala Cys Ala Cys Asp Gln Lys Pro Cys
65                  70                  75                  80

Ser Cys Ser Lys Val Asp Val Asn Tyr Ala Phe Leu His Ala Thr Asp

                85                  90                  95

Leu Leu Pro Ala Cys Asp Gly Glu Arg Pro Thr Leu Ala Phe Leu Gln
            100                 105                 110

Asp Val Met Asn Ile Leu Leu Gln Tyr Val Val Lys Ser Phe Asp Arg
        115                 120                 125

Ser Thr Lys Val Ile Asp Phe His Tyr Pro Asn Glu Leu Leu Gln Glu
    130                 135                 140

Tyr Asn Trp Glu Leu Ala Asp Gln Pro Gln Asn Leu Glu Glu Ile Leu
145                 150                 155                 160

Met His Cys Gln Thr Thr Leu Lys Tyr Ala Ile Lys Thr Gly His Pro
                165                 170                 175

Arg Tyr Phe Asn Gln Leu Ser Thr Gly Leu Asp Met Val Gly Leu Ala
            180                 185                 190

Ala Asp Trp Leu Thr Ser Thr Ala Asn Thr Asn Met Phe Thr Tyr Glu
        195                 200                 205

Ile Ala Pro Val Phe Val Leu Leu Glu Tyr Val Thr Leu Lys Lys Met
    210                 215                 220
Arg Glu Ile Ile Gly Trp Pro Gly Gly Ser Gly Asp Gly Ile Phe Ser
225                 230                 235                 240
Pro Gly Gly Ala Ile Ser Asn Met Tyr Ala Met Met Ile Ala Arg Phe
                245                 250                 255
Lys Met Phe Pro Glu Val Lys Glu Lys Gly Met Ala Ala Leu Pro Arg
            260                 265                 270
Leu Ile Ala Phe Thr Ser Glu His Ser His Phe Ser Leu Lys Lys Gly
        275                 280                 285
Ala Ala Ala Leu Gly Ile Gly Thr Asp Ser Val Ile Leu Ile Lys Cys
    290                 295                 300
Asp Glu Arg Gly Lys Met Ile Pro Ser Asp Leu Glu Arg Arg Ile Leu
305                 310                 315                 320

Glu Ala Lys Gln Lys Gly Phe Val Pro Phe Leu Val Ser Ala Thr Ala
                325                 330                 335

Gly Thr Thr Val Tyr Gly Ala Phe Asp Pro Leu Leu Ala Val Ala Asp
            340                 345                 350

Ile Cys Lys Lys Tyr Lys Ile Trp Met His Val Asp Ala Ala Trp Gly
        355                 360                 365

Gly Gly Leu Leu Met Ser Arg Lys His Lys Trp Lys Leu Ser Gly Val
    370                 375                 380

Glu Arg Ala Asn Ser Val Thr Trp Asn Pro His Lys Met Met Gly Val
385                 390                 395                 400

Pro Leu Gln Cys Ser Ala Leu Leu Val Arg Glu Glu Gly Leu Met Gln
                405                 410                 415

Asn Cys Asn Gln Met His Ala Ser Tyr Leu Phe Gln Gln Asp Lys His
            420                 425                 430

Tyr Asp Leu Ser Tyr Asp Thr Gly Asp Lys Ala Leu Gln Cys Gly Arg
        435                 440                 445

His Val Asp Val Phe Lys Leu Trp Leu Met Trp Arg Ala Lys Gly Thr
    450                 455                 460

Thr Gly Phe Glu Ala His Val Asp Lys Cys Leu Glu Leu Ala Glu Tyr
465                 470                 475                 480

Leu Tyr Asn Ile Ile Lys Asn Arg Glu Gly Tyr Glu Met Val Phe Asp
                485                 490                 495

Gly Lys Pro Gln His Thr Asn Val Cys Phe Trp Tyr Ile Pro Pro Ser
            500                 505                 510

Leu Arg Thr Leu Glu Asp Asn Glu Glu Arg Met Ser Arg Leu Ser Lys
        515                 520                 525

Val Ala Pro Val Ile Lys Ala Arg Met Met Glu Tyr Gly Thr Thr Met
    530                 535                 540

Val Ser Tyr Gln Pro Leu Gly Asp Lys Val Asn Phe Phe Arg Met Val

545                 550                 555                 560

Ile Ser Asn Pro Ala Ala Thr His Gln Asp Ile Asp Phe Leu Ile Glu
                565                 570                 575

Glu Ile Glu Arg Leu Gly Gln Asp Leu His His His His His His
            580                 585                 590

<210>  3
<211>  1491
<212>  DNA
<213>  Human GAD65 1-95deletion

<220>
<221>  CDS
<222>  (1)..(1488)
<223>

<400>  3
atg gtg ctg ccg gcg tgt gat gga gaa agg ccc act ttg gcg ttt ctg        48
Met Val Leu Pro Ala Cys Asp Gly Glu Arg Pro Thr Leu Ala Phe Leu
1               5                   10                  15

caa gat gtt atg aac att tta ctt cag tat gtg gtg aaa agt ttc gat        96
Gln Asp Val Met Asn Ile Leu Leu Gln Tyr Val Val Lys Ser Phe Asp
            20                  25                  30

aga tca acc aaa gtg att gat ttc cat tat cct aat gag ctt ctc caa 144
Arg Ser Thr Lys Val Ile Asp Phe His Tyr Pro Asn Glu Leu Leu Gln
        35                  40                  45

gaa tat aat tgg gaa ttg gca gac caa cca caa aat ttg gag gaa att 192
Glu Tyr Asn Trp Glu Leu Ala Asp Gln Pro Gln Asn Leu Glu Glu Ile
    50                  55                  60

ttg atg cat tgc caa aca act cta aaa tat gca att aaa aca ggg cat 240
Leu Met His Cys Gln Thr Thr Leu Lys Tyr Ala Ile Lys Thr Gly His
65                  70                  75                  80

cct aga tac ttc aat caa ctt tct act ggt ttg gat atg gtt gga tta 288
Pro Arg Tyr Phe Asn Gln Leu Ser Thr Gly Leu Asp Met Val Gly Leu
                85                  90                  95

gca gca gac tgg ctg aca tca aca gca aat act aac atg ttc acc tat 336
Ala Ala Asp Trp Leu Thr Ser Thr Ala Asn Thr Asn Met Phe Thr Tyr
            100                 105                 110

gaa att gct cca gta ttt gtg ctt ttg gaa tat gtc aca cta aag aaa 384
Glu Ile Ala Pro Val Phe Val Leu Leu Glu Tyr Val Thr Leu Lys Lys
        115                 120                 125

atg aga gaa atc att ggc tgg cca ggg ggc tct ggc gat ggg ata ttt 432
Met Arg Glu Ile Ile Gly Trp Pro Gly Gly Ser Gly Asp Gly Ile Phe
    130                 135                 140

tct ccc ggt ggc gcc ata tct aac atg tat gcc atg atg atc gca cgc 480
Ser Pro Gly Gly Ala Ile Ser Asn Met Tyr Ala Met Met Ile Ala Arg
145                 150                 155                 160

ttt aag atg ttc cca gaa gtc aag gag aaa gga atg gct gct ctt ccc 528
Phe Lys Met Phe Pro Glu Val Lys Glu Lys Gly Met Ala Ala Leu Pro
                165                 170                 175

agg ctc att gcc ttc acg tct gaa cat agt cat ttt tct ctc aag aag 576
Arg Leu Ile Ala Phe Thr Ser Glu His Ser His Phe Ser Leu Lys Lys
            180                 185                 190

gga gct gca gcc tta ggg att gga aca gac agc gtg att ctg att aaa 624
Gly Ala Ala Ala Leu Gly Ile Gly Thr Asp Ser Val Ile Leu Ile Lys
        195                 200                 205

tgt gat gag aga ggg aaa atg att cca tct gat ctt gaa aga agg att 672
Cys Asp Glu Arg Gly Lys Met Ile Pro Ser Asp Leu Glu Arg Arg Ile
    210                 215                 220

ctt gaa gcc aaa cag aaa ggg ttt gtt cct ttc ctc gtg agt gcc aca 720
Leu Glu Ala Lys Gln Lys Gly Phe Val Pro Phe Leu Val Ser Ala Thr
225                 230                 235                 240

gct gga acc acc gtg tac gga gca ttt gac ccc ctc tta gct gtc gct 768
Ala Gly Thr Thr Val Tyr Gly Ala Phe Asp Pro Leu Leu Ala Val Ala
                245                 250                 255

gac att tgc aaa aag tat aag atc tgg atg cat gtg gat gca gct tgg 816

Asp Ile Cys Lys Lys Tyr Lys Ile Trp Met His Val Asp Ala Ala Trp
            260                 265                 270

ggt ggg gga tta ctg atg tcc cga aaa cac aag tgg aaa ctg agt ggc 864
Gly Gly Gly Leu Leu Met Ser Arg Lys His Lys Trp Lys Leu Ser Gly
        275                 280                 285

gtg gag agg gcc aac tct gtg acg tgg aat cca cac aag atg atg gga 912
Val Glu Arg Ala Asn Ser Val Thr Trp Asn Pro His Lys Met Met Gly
    290                 295                 300

gtc cct ttg cag tgc tct gct ctc ctg gtt aga gaa gag gga ttg atg 960
Val Pro Leu Gln Cys Ser Ala Leu Leu Val Arg Glu Glu Gly Leu Met
305                 310                 315                 320

cag aat tgc aac caa atg cat gcc tcc tac ctc ttt cag caa gat aaa 1008
Gln Asn Cys Asn Gln Met His Ala Ser Tyr Leu Phe Gln Gln Asp Lys
                325                 330                 335

cat tat gac ctg tcc tat gac act gga gac aag gcc tta cag tgc gga 1056
His Tyr Asp Leu Ser Tyr Asp Thr Gly Asp Lys Ala Leu Gln Cys Gly
            340                 345                 350

cgc cac gtt gat gtt ttt aaa cta tgg ctg atg tgg agg gca aag ggg 1104
Arg His Val Asp Val Phe Lys Leu Trp Leu Met Trp Arg Ala Lys Gly
        355                 360                 365

act acc ggg ttt gaa gcg cat gtt gat aaa tgt ttg gag ttg gca gag 1152
Thr Thr Gly Phe Glu Ala His Val Asp Lys Cys Leu Glu Leu Ala Glu

    370                 375                 380

tat tta tac aac atc ata aaa aac cga gaa gga tat gag atg gtg ttt 1200
Tyr Leu Tyr Asn Ile Ile Lys Asn Arg Glu Gly Tyr Glu Met Val Phe
385                 390                 395                 400

gat ggg aag cct cag cac aca aat gtc tgc ttc tgg tac att cct cca 1248
Asp Gly Lys Pro Gln His Thr Asn Val Cys Phe Trp Tyr Ile Pro Pro
                405                 410                 415

agc ttg cgt act ctg gaa gac aat gaa gag aga atg agt cgc ctc tcg 1296
Ser Leu Arg Thr Leu Glu Asp Asn Glu Glu Arg Met Ser Arg Leu Ser
            420                 425                 430

aag gtg gct cca gtg att aaa gcc aga atg atg gag tat gga acc aca 1344
Lys Val Ala Pro Val Ile Lys Ala Arg Met Met Glu Tyr Gly Thr Thr
        435                 440                 445

atg gtc agc tac caa ccc ttg gga gac aag gtc aat ttc ttc cgc atg 1392
Met Val Ser Tyr Gln Pro Leu Gly Asp Lys Val Asn Phe Phe Arg Met
    450                 455                 460

gtc atc tca aac cca gcg gca act cac caa gac att gac ttc ctg att 1440
Val Ile Ser Asn Pro Ala Ala Thr His Gln Asp Ile Asp Phe Leu Ile
465                 470                 475                 480

gaa gaa ata gaa cgc ctt gga caa gat tta cac cac cac cac cac cac 1488
Glu Glu Ile Glu Arg Leu Gly Gln Asp Leu His His His His His His
                485                 490                 495

taa                                                                    1491

<210>  4
<211>  496
<212>  PRT
<213>  Human GAD65 1-95deletion

<400>  4

Met Val Leu Pro Ala Cys Asp Gly Glu Arg Pro Thr Leu Ala Phe Leu
1               5                   10                  15

Gln Asp Val Met Asn Ile Leu Leu Gln Tyr Val Val Lys Ser Phe Asp
            20                  25                  30

Arg Ser Thr Lys Val Ile Asp Phe His Tyr Pro Asn Glu Leu Leu Gln
        35                  40                  45

Glu Tyr Asn Trp Glu Leu Ala Asp Gln Pro Gln Asn Leu Glu Glu Ile
    50                  55                  60

Leu Met His Cys Gln Thr Thr Leu Lys Tyr Ala Ile Lys Thr Gly His
65                  70                  75                  80

Pro Arg Tyr Phe Asn Gln Leu Ser Thr Gly Leu Asp Met Val Gly Leu
                85                  90                  95

Ala Ala Asp Trp Leu Thr Ser Thr Ala Asn Thr Asn Met Phe Thr Tyr
            100                 105                 110

Glu Ile Ala Pro Val Phe Val Leu Leu Glu Tyr Val Thr Leu Lys Lys
        115                 120                 125

Met Arg Glu Ile Ile Gly Trp Pro Gly Gly Ser Gly Asp Gly Ile Phe
    130                 135                 140

Ser Pro Gly Gly Ala Ile Ser Asn Met Tyr Ala Met Met Ile Ala Arg
145                 150                 155                 160

Phe Lys Met Phe Pro Glu Val Lys Glu Lys Gly Met Ala Ala Leu Pro
                165                 170                 175

Arg Leu Ile Ala Phe Thr Ser Glu His Ser His Phe Ser Leu Lys Lys
            180                 185                 190

Gly Ala Ala Ala Leu Gly Ile Gly Thr Asp Ser Val Ile Leu Ile Lys
        195                 200                 205

Cys Asp Glu Arg Gly Lys Met Ile Pro Ser Asp Leu Glu Arg Arg Ile
    210                 215                 220

Leu Glu Ala Lys Gln Lys Gly Phe Val Pro Phe Leu Val Ser Ala Thr
225                 230                 235                 240

Ala Gly Thr Thr Val Tyr Gly Ala Phe Asp Pro Leu Leu Ala Val Ala
                245                 250                 255

Asp Ile Cys Lys Lys Tyr Lys Ile Trp Met His Val Asp Ala Ala Trp
            260                 265                 270

Gly Gly Gly Leu Leu Met Ser Arg Lys His Lys Trp Lys Leu Ser Gly
        275                 280                 285

Val Glu Arg Ala Asn Ser Val Thr Trp Asn Pro His Lys Met Met Gly
    290                 295                 300

Val Pro Leu Gln Cys Ser Ala Leu Leu Val Arg Glu Glu Gly Leu Met
305                 310                 315                 320

Gln Asn Cys Asn Gln Met His Ala Ser Tyr Leu Phe Gln Gln Asp Lys
                325                 330                 335

His Tyr Asp Leu Ser Tyr Asp Thr Gly Asp Lys Ala Leu Gln Cys Gly
            340                 345                 350

Arg His Val Asp Val Phe Lys Leu Trp Leu Met Trp Arg Ala Lys Gly
        355                 360                 365

Thr Thr Gly Phe Glu Ala His Val Asp Lys Cys Leu Glu Leu Ala Glu
    370                 375                 380

Tyr Leu Tyr Asn Ile Ile Lys Asn Arg Glu Gly Tyr Glu Met Val Phe
385                 390                 395                 400

Asp Gly Lys Pro Gln His Thr Asn Val Cys Phe Trp Tyr Ile Pro Pro
                405                 410                 415

Ser Leu Arg Thr Leu Glu Asp Asn Glu Glu Arg Met Ser Arg Leu Ser

            420                 425                 430

Lys Val Ala Pro Val Ile Lys Ala Arg Met Met Glu Tyr Gly Thr Thr
        435                 440                 445

Met Val Ser Tyr Gln Pro Leu Gly Asp Lys Val Asn Phe Phe Arg Met
    450                 455                 460

Val Ile Ser Asn Pro Ala Ala Thr His Gln Asp Ile Asp Phe Leu Ile
465                 470                 475                 480

Glu Glu Ile Glu Arg Leu Gly Gln Asp Leu His His His His His His
                485                 490                 495

<210> 5
<211> 1215
<212> DNA
<213> human GAD65 1-187deletion mutant(F52)

<220>
<221> CDS
<222> (1)..(1212)
<223>

<400> 5

atg gtt gga tta gca gca gac tgg ctg aca tca aca gca aat act aac 48
Met Val Gly Leu Ala Ala Asp Trp Leu Thr Ser Thr Ala Asn Thr Asn
1               5                   10                  15

atg ttc acc tat gaa att gct cca gta ttt gtg ctt ttg gaa tat gtc 96
Met Phe Thr Tyr Glu Ile Ala Pro Val Phe Val Leu Leu Glu Tyr Val
            20                  25                  30

aca cta aag aaa atg aga gaa atc att ggc tgg cca ggg ggc tct ggc 144
Thr Leu Lys Lys Met Arg Glu Ile Ile Gly Trp Pro Gly Gly Ser Gly
        35                  40                  45

gat ggg ata ttt tct ccc ggt ggc gcc ata tct aac atg tat gcc atg 192
Asp Gly Ile Phe Ser Pro Gly Gly Ala Ile Ser Asn Met Tyr Ala Met
    50                  55                  60

atg atc gca cgc ttt aag atg ttc cca gaa gtc aag gag aaa gga atg 240
Met Ile Ala Arg Phe Lys Met Phe Pro Glu Val Lys Glu Lys Gly Met
65                  70                  75                  80

gct gct ctt ccc agg ctc att gcc ttc acg tct gaa cat agt cat ttt 288
Ala Ala Leu Pro Arg Leu Ile Ala Phe Thr Ser Glu His Ser His Phe
                85                  90                  95

tct ctc aag aag gga gct gca gcc tta ggg att gga aca gac agc gtg 336
Ser Leu Lys Lys Gly Ala Ala Ala Leu Gly Ile Gly Thr Asp Ser Val
            100                 105                 110

att ctg att aaa tgt gat gag aga ggg aaa atg att cca tct gat ctt 384
Ile Leu Ile Lys Cys Asp Glu Arg Gly Lys Met Ile Pro Ser Asp Leu
        115                 120                 125

gaa aga agg att ctt gaa gcc aaa cag aaa ggg ttt gtt cct ttc ctc 432
Glu Arg Arg Ile Leu Glu Ala Lys Gln Lys Gly Phe Val Pro Phe Leu
    130                 135                 140

gtg agt gcc aca gct gga acc acc gtg tac gga gca ttt gac ccc ctc 480
Val Ser Ala Thr Ala Gly Thr Thr Val Tyr Gly Ala Phe Asp Pro Leu
145                 150                 155                 160

tta gct gtc gct gac att tgc aaa aag tat aag atc tgg atg cat gtg 528
Leu Ala Val Ala Asp Ile Cys Lys Lys Tyr Lys Ile Trp Met His Val
                165                 170                 175

gat gca gct tgg ggt ggg gga tta ctg atg tcc cga aaa cac aag tgg 576
Asp Ala Ala Trp Gly Gly Gly Leu Leu Met Ser Arg Lys His Lys Trp
            180                 185                 190

aaa ctg agt ggc gtg gag agg gcc aac tct gtg acg tgg aat cca cac 624
Lys Leu Ser Gly Val Glu Arg Ala Asn Ser Val Thr Trp Asn Pro His
        195                 200                 205

aag atg atg gga gtc cct ttg cag tgc tct gct ctc ctg gtt aga gaa 672
Lys Met Met Gly Val Pro Leu Gln Cys Ser Ala Leu Leu Val Arg Glu
    210                 215                 220

ggg gga ttg atg cag aat tgc aac caa atg cat gcc tcc tac ctc ttt 720

Gly Gly Leu Met Gln Asn Cys Asn Gln Met His Ala Ser Tyr Leu Phe
225                 230                 235                 240

cag caa gat aaa cat tat gac ctg tcc tat gac act gga gac aag gcc 768
Gln Gln Asp Lys His Tyr Asp Leu Ser Tyr Asp Thr Gly Asp Lys Ala
                245                 250                 255

tta cag tgc gga cgc cac gtt gat gtt ttt aaa cta tgg ctg atg tgg 816
Leu Gln Cys Gly Arg His Val Asp Val Phe Lys Leu Trp Leu Met Trp
            260                 265                 270

agg gca aag ggg act acc ggg ttt gaa gcg cat gtt gat aaa tgt ttg 864
Arg Ala Lys Gly Thr Thr Gly Phe Glu Ala His Val Asp Lys Cys Leu
        275                 280                 285

gag ttg gca gag tat tta tac aac atc ata aaa aac cga gaa gga tat 912
Glu Leu Ala Glu Tyr Leu Tyr Asn Ile Ile Lys Asn Arg Glu Gly Tyr
    290                 295                 300

gag atg gtg ttt gat ggg aag cct cag cac aca aat gtc tgc ttc tgg 960
Glu Met Val Phe Asp Gly Lys Pro Gln His Thr Asn Val Cys Phe Trp
305                 310                 315                 320

tac att cct cca agc ttg cgt act ctg gaa gac aat gaa gag aga atg 1008
Tyr Ile Pro Pro Ser Leu Arg Thr Leu Glu Asp Asn Glu Glu Arg Met
                325                 330                 335

agt cgc ctc tcg aag gtg gct cca gtg att aaa gcc aga atg atg gag 1056
Ser Arg Leu Ser Lys Val Ala Pro Val Ile Lys Ala Arg Met Met Glu

            340                 345                 350

tat gga acc aca atg gtc agc tac caa ccc ttg gga gac aag gtc aat 1104
Tyr Gly Thr Thr Met Val Ser Tyr Gln Pro Leu Gly Asp Lys Val Asn
        355                 360                 365

ttc ttc cgc atg gtc atc tca aac cca gcg gca act cac caa gac att 1152
Phe Phe Arg Met Val Ile Ser Asn Pro Ala Ala Thr His Gln Asp Ile
    370                 375                 380

gac ttc ctg att gaa gaa ata gaa cgc ctt gga caa gat tta cac cac 1200
Asp Phe Leu Ile Glu Glu Ile Glu Arg Leu Gly Gln Asp Leu His His
385                 390                 395                 400

cac cac cac cac taa 1215
His His His His

<210> 6
<211> 404
<212> PRT
<213> human GAD65 1-187deletion mutant(F52)

<400> 6

Met Val Gly Leu Ala Ala Asp Trp Leu Thr Ser Thr Ala Asn Thr Asn
1               5                   10                  15

Met Phe Thr Tyr Glu Ile Ala Pro Val Phe Val Leu Leu Glu Tyr Val
            20                  25                  30
Thr Leu Lys Lys Met Arg Glu Ile Ile Gly Trp Pro Gly Gly Ser Gly
        35                  40                  45
Asp Gly Ile Phe Ser Pro Gly Gly Ala Ile Ser Asn Met Tyr Ala Met
    50                  55                  60
Met Ile Ala Arg Phe Lys Met Phe Pro Glu Val Lys Glu Lys Gly Met
65                  70                  75                  80
Ala Ala Leu Pro Arg Leu Ile Ala Phe Thr Ser Glu His Ser His Phe
                85                  90                  95
Ser Leu Lys Lys Gly Ala Ala Ala Leu Gly Ile Gly Thr Asp Ser Val
            100                 105                 110
Ile Leu Ile Lys Cys Asp Glu Arg Gly Lys Met Ile Pro Ser Asp Leu
        115                 120                 125

Glu Arg Arg Ile Leu Glu Ala Lys Gln Lys Gly Phe Val Pro Phe Leu
    130                 135                 140

Val Ser Ala Thr Ala Gly Thr Thr Val Tyr Gly Ala Phe Asp Pro Leu
145                 150                 155                 160

Leu Ala Val Ala Asp Ile Cys Lys Lys Tyr Lys Ile Trp Met His Val
                165                 170                 175

Asp Ala Ala Trp Gly Gly Gly Leu Leu Met Ser Arg Lys His Lys Trp
            180                 185                 190

Lys Leu Ser Gly Val Glu Arg Ala Asn Ser Val Thr Trp Asn Pro His
        195                 200                 205

Lys Met Met Gly Val Pro Leu Gln Cys Ser Ala Leu Leu Val Arg Glu
    210                 215                 220

Gly Gly Leu Met Gln Asn Cys Asn Gln Met His Ala Ser Tyr Leu Phe
225                 230                 235                 240

Gln Gln Asp Lys His Tyr Asp Leu Ser Tyr Asp Thr Gly Asp Lys Ala

                245                 250                 255

Leu Gln Cys Gly Arg His Val Asp Val Phe Lys Leu Trp Leu Met Trp
            260                 265                 270

Arg Ala Lys Gly Thr Thr Gly Phe Glu Ala His Val Asp Lys Cys Leu
        275                 280                 285

Glu Leu Ala Glu Tyr Leu Tyr Asn Ile Ile Lys Asn Arg Glu Gly Tyr
    290                 295                 300

Glu Met Val Phe Asp Gly Lys Pro Gln His Thr Asn Val Cys Phe Trp
305                 310                 315                 320

Tyr Ile Pro Pro Ser Leu Arg Thr Leu Glu Asp Asn Glu Glu Arg Met
                325                 330                 335

Ser Arg Leu Ser Lys Val Ala Pro Val Ile Lys Ala Arg Met Met Glu
            340                 345                 350

Tyr Gly Thr Thr Met Val Ser Tyr Gln Pro Leu Gly Asp Lys Val Asn
        355                 360                 365

Phe Phe Arg Met Val Ile Ser Asn Pro Ala Ala Thr His Gln Asp Ile
    370                 375                 380

Asp Phe Leu Ile Glu Glu Ile Glu Arg Leu Gly Gln Asp Leu His His
385                 390                 395                 400

His His His His

<210> 7
<211> 1215
<212> DNA
<213> human GAD65 1-187deletion mutant(F68)

<220>
<221> CDS
<222> (1)..(1212)
<223>

<400> 7
atg gtt gga tta gca gca gac tgg ctg aca tca aca gca aat act aac 48
Met Val Gly Leu Ala Ala Asp Trp Leu Thr Ser Thr Ala Asn Thr Asn
1               5                   10                  15

atg ttc acc tat gaa att gct cca gta ttt gtg ctt ttg gaa tat gtc 96
Met Phe Thr Tyr Glu Ile Ala Pro Val Phe Val Leu Leu Glu Tyr Val
            20                  25                  30

aca cta aag aaa atg aga gaa atc att ggc tgg cca ggg ggc tct ggc 144
Thr Leu Lys Lys Met Arg Glu Ile Ile Gly Trp Pro Gly Gly Ser Gly
        35                  40                  45

gat ggg ata ttt tct ccc ggt ggc gcc ata tct aac atg tat gcc atg 192
Asp Gly Ile Phe Ser Pro Gly Gly Ala Ile Ser Asn Met Tyr Ala Met
    50                  55                  60

atg atc gca cgc ttt aag atg ttc cca gaa gtc aag gag aaa gga atg 240
Met Ile Ala Arg Phe Lys Met Phe Pro Glu Val Lys Glu Lys Gly Met
65                  70                  75                  80

gct gct ctt ccc agg ctc att gcc ttc acg tct gaa cat agt cat ttt 288
Ala Ala Leu Pro Arg Leu Ile Ala Phe Thr Ser Glu His Ser His Phe
                85                  90                  95

tct ctc aag aag gga gct gca gcc tta ggg att gga aca gac agc gtg 336
Ser Leu Lys Lys Gly Ala Ala Ala Leu Gly Ile Gly Thr Asp Ser Val
            100                 105                 110

att ctg att aaa tgt gat gag aga ggg aaa atg att cca ttt gat ctt 384
Ile Leu Ile Lys Cys Asp Glu Arg Gly Lys Met Ile Pro Phe Asp Leu
        115                 120                 125

gaa aga agg att ctt gaa gcc aaa cag aaa ggg ttt gtt cct ttc ctc 432

Glu Arg Arg Ile Leu Glu Ala Lys Gln Lys Gly Phe Val Pro Phe Leu
    130                 135                 140

gtg agt gcc aca gct gga acc acc gtg tac gga gca ttt gac ccc ctc 480
Val Ser Ala Thr Ala Gly Thr Thr Val Tyr Gly Ala Phe Asp Pro Leu
145                 150                 155                 160

tta gct gtc gct gac att tgc aaa aag tat aag atc tgg atg cat gtg 528
Leu Ala Val Ala Asp Ile Cys Lys Lys Tyr Lys Ile Trp Met His Val
                165                 170                 175

gat gca gct tgg ggt ggg gga tta ctg atg tcc cga aaa cac aag tgg 576
Asp Ala Ala Trp Gly Gly Gly Leu Leu Met Ser Arg Lys His Lys Trp
            180                 185                 190

aaa ctg agt ggc gtg gag agg gcc aac tct gtg acg tgg aat cca cac 624
Lys Leu Ser Gly Val Glu Arg Ala Asn Ser Val Thr Trp Asn Pro His
        195                 200                 205

aag atg atg gga gtc cct ttg cag tgc tct gct ctc ctg gtt aga gaa 672
Lys Met Met Gly Val Pro Leu Gln Cys Ser Ala Leu Leu Val Arg Glu
    210                 215                 220

gag gga ttg atg cag aat tgc aac caa atg cat gcc tcc tac ctc ttt 720
Glu Gly Leu Met Gln Asn Cys Asn Gln Met His Ala Ser Tyr Leu Phe
225                 230                 235                 240

cag caa gat aaa cat tat gac ctg tcc tat gac act gga gac aag gcc 768
Gln Gln Asp Lys His Tyr Asp Leu Ser Tyr Asp Thr Gly Asp Lys Ala

                245                 250                 255

tta cag tgc gga cgc cac gtt gat gtt ttt aaa cta tgg ctg atg tgg 816
Leu Gln Cys Gly Arg His Val Asp Val Phe Lys Leu Trp Leu Met Trp
            260                 265                 270

agg gca aag ggg act acc ggg ttt gaa gcg cat gtt gat aaa tgt ttg 864
Arg Ala Lys Gly Thr Thr Gly Phe Glu Ala His Val Asp Lys Cys Leu
        275                 280                 285

gag ttg gca gag tat tta tac aac atc ata aaa aac cga gaa gga tat 912
Glu Leu Ala Glu Tyr Leu Tyr Asn Ile Ile Lys Asn Arg Glu Gly Tyr
    290                 295                 300

gag atg gtg ttt gat ggg aag cct cag cac aca aat gtc tgc ttc tgg 960
Glu Met Val Phe Asp Gly Lys Pro Gln His Thr Asn Val Cys Phe Trp
305                 310                 315                 320

tac att cct cca agc ttg cgt act ctg gaa gac aat gaa gag aga atg 1008
Tyr Ile Pro Pro Ser Leu Arg Thr Leu Glu Asp Asn Glu Glu Arg Met
                325                 330                 335

agt cgc ctc tcg aag gtg gct cca gtg att aaa gcc aga atg atg gag 1056
Ser Arg Leu Ser Lys Val Ala Pro Val Ile Lys Ala Arg Met Met Glu
            340                 345                 350

tat gga acc aca atg gtc agc tac caa ccc ttg gga gac aag gtc aat 1104
Tyr Gly Thr Thr Met Val Ser Tyr Gln Pro Leu Gly Asp Lys Val Asn
        355                 360                 365

ttc ttc cgc atg gtc atc tca aac cca gcg gca act cac caa gac att 1152
Phe Phe Arg Met Val Ile Ser Asn Pro Ala Ala Thr His Gln Asp Ile
    370                 375                 380

gac ttc ctg att gaa gaa ata gaa cgc ctt gga caa gat tta cac cac 1200
Asp Phe Leu Ile Glu Glu Ile Glu Arg Leu Gly Gln Asp Leu His His
385                 390                 395                 400

cac cac cac cac taa 1215
His His His His

<210> 8
<211> 404
<212> PRT
<213> human GAD65 1-187deletion mutant (F68)

<400> 8

Met Val Gly Leu Ala Ala Asp Trp Leu Thr Ser Thr Ala Asn Thr Asn
1               5                   10                  15

Met Phe Thr Tyr Glu Ile Ala Pro Val Phe Val Leu Leu Glu Tyr Val
            20                  25                  30

Thr Leu Lys Lys Met Arg Glu Ile Ile Gly Trp Pro Gly Gly Ser Gly
        35                  40                  45

Asp Gly Ile Phe Ser Pro Gly Gly Ala Ile Ser Asn Met Tyr Ala Met
    50                  55                  60

Met Ile Ala Arg Phe Lys Met Phe Pro Glu Val Lys Glu Lys Gly Met
65                  70                  75                  80

Ala Ala Leu Pro Arg Leu Ile Ala Phe Thr Ser Glu His Ser His Phe
                85                  90                  95

Ser Leu Lys Lys Gly Ala Ala Ala Leu Gly Ile Gly Thr Asp Ser Val
            100                 105                 110

Ile Leu Ile Lys Cys Asp Glu Arg Gly Lys Met Ile Pro Phe Asp Leu
        115                 120                 125

Glu Arg Arg Ile Leu Glu Ala Lys Gln Lys Gly Phe Val Pro Phe Leu
    130                 135                 140

Val Ser Ala Thr Ala Gly Thr Thr Val Tyr Gly Ala Phe Asp Pro Leu

145                    150                    155                    160

Leu Ala Val Ala Asp Ile Cys Lys Lys Tyr Lys Ile Trp Met His Val
                    165                    170                    175

Asp Ala Ala Trp Gly Gly Gly Leu Leu Met Ser Arg Lys His Lys Trp
                180                    185                    190

Lys Leu Ser Gly Val Glu Arg Ala Asn Ser Val Thr Trp Asn Pro His
            195                    200                    205

Lys Met Met Gly Val Pro Leu Gln Cys Ser Ala Leu Leu Val Arg Glu
        210                    215                    220

Glu Gly Leu Met Gln Asn Cys Asn Gln Met His Ala Ser Tyr Leu Phe
225                    230                    235                    240

Gln Gln Asp Lys His Tyr Asp Leu Ser Tyr Asp Thr Gly Asp Lys Ala
                    245                    250                    255

Leu Gln Cys Gly Arg His Val Asp Val Phe Lys Leu Trp Leu Met Trp
                260                    265                    270

Arg Ala Lys Gly Thr Thr Gly Phe Glu Ala His Val Asp Lys Cys Leu
        275                 280                 285

Glu Leu Ala Glu Tyr Leu Tyr Asn Ile Ile Lys Asn Arg Glu Gly Tyr
    290                 295                 300

Glu Met Val Phe Asp Gly Lys Pro Gln His Thr Asn Val Cys Phe Trp
305                 310                 315                 320

Tyr Ile Pro Pro Ser Leu Arg Thr Leu Glu Asp Asn Glu Glu Arg Met
                325                 330                 335

Ser Arg Leu Ser Lys Val Ala Pro Val Ile Lys Ala Arg Met Met Glu
            340                 345                 350

Tyr Gly Thr Thr Met Val Ser Tyr Gln Pro Leu Gly Asp Lys Val Asn
        355                 360                 365

Phe Phe Arg Met Val Ile Ser Asn Pro Ala Ala Thr His Gln Asp Ile
    370                 375                 380

Asp Phe Leu Ile Glu Glu Ile Glu Arg Leu Gly Gln Asp Leu His His
385                 390                 395                 400

His His His His

<210> 9
<211> 1215
<212> DNA
<213> human GAD65 1-187deletion mutant (F1)

<220>
<221> CDS
<222> (1)..(1212)
<223>

<400> 9
atg gtt gga tta gca gca gac tgg ctg aca tca aca gca aat act aac      48
Met Val Gly Leu Ala Ala Asp Trp Leu Thr Ser Thr Ala Asn Thr Asn
1               5                   10                  15

atg ttc acc tat gaa att gct cca gta ttt gtg ctt ttg gaa tat gtc      96
Met Phe Thr Tyr Glu Ile Ala Pro Val Phe Val Leu Leu Glu Tyr Val
            20                  25                  30

aca cta aag aaa atg aga gaa atc att ggc tgg cca ggg ggc tct ggc      144

Thr Leu Lys Lys Met Arg Glu Ile Ile Gly Trp Pro Gly Gly Ser Gly
        35                  40                  45

gat ggg ata ttt tct ccc ggt ggc gcc ata tct aac atg tat gcc atg 192
Asp Gly Ile Phe Ser Pro Gly Gly Ala Ile Ser Asn Met Tyr Ala Met
    50                  55                  60

atg atc gca cgc ttt aag atg ttc cca gaa gtc aag gag aaa gga atg 240
Met Ile Ala Arg Phe Lys Met Phe Pro Glu Val Lys Glu Lys Gly Met
65                  70                  75                  80

gct gct ctt ccc agg ctc att gcc ttc acg tct gaa cat agt cat ttt 288
Ala Ala Leu Pro Arg Leu Ile Ala Phe Thr Ser Glu His Ser His Phe
                85                  90                  95

tct ctc aag aag gga gct gca gcc tta ggg att gga aca gac agc gtg 336
Ser Leu Lys Lys Gly Ala Ala Ala Leu Gly Ile Gly Thr Asp Ser Val
            100                 105                 110

att ctg att aaa tgt gat gag aga ggg aaa atg att cca tct gat ctt 384
Ile Leu Ile Lys Cys Asp Glu Arg Gly Lys Met Ile Pro Ser Asp Leu
        115                 120                 125

gaa aga agg att ctt gaa gcc aaa cag aaa ggg ttt gtt cct ttc ctc 432
Glu Arg Arg Ile Leu Glu Ala Lys Gln Lys Gly Phe Val Pro Phe Leu
    130                 135                 140

gtg agt gcc aca gct gga acc acc gtg tac gga gca ttt gac ccc ctc 480
Val Ser Ala Thr Ala Gly Thr Thr Val Tyr Gly Ala Phe Asp Pro Leu

145                 150                 155                 160

tta gct gtc gct gac att tgc aaa aag tat aag atc tgg ata cat gtg       528
Leu Ala Val Ala Asp Ile Cys Lys Lys Tyr Lys Ile Trp Ile His Val
                165                 170                 175

gat gca gct tgg ggt ggg gga tta ctg atg tcc cga aaa cac aag tgg       576
Asp Ala Ala Trp Gly Gly Gly Leu Leu Met Ser Arg Lys His Lys Trp
            180                 185                 190

aaa ctg agt ggc gtg gag agg gcc aac tct gtg acg tgg aat cca cac       624
Lys Leu Ser Gly Val Glu Arg Ala Asn Ser Val Thr Trp Asn Pro His
        195                 200                 205

aag atg atg gga gtc cct ttg cag tgc tct gct ctc ctg gtt aga gaa       672
Lys Met Met Gly Val Pro Leu Gln Cys Ser Ala Leu Leu Val Arg Glu
    210                 215                 220

gag gga ttg atg cag aat tgc aac caa atg cat gcc tcc tac ctc ttt       720
Glu Gly Leu Met Gln Asn Cys Asn Gln Met His Ala Ser Tyr Leu Phe
225                 230                 235                 240

cag caa gat aaa cat tat gac ctg tcc tat gac act gga gac aag gcc       768
Gln Gln Asp Lys His Tyr Asp Leu Ser Tyr Asp Thr Gly Asp Lys Ala
                245                 250                 255

tta cag tgc gga cgc cac gtt gat gtt ttt aaa cta tgg ctg atg tgg       816
Leu Gln Cys Gly Arg His Val Asp Val Phe Lys Leu Trp Leu Met Trp
            260                 265                 270

agg gca aag ggg act acc ggg tct gaa gcg cat gtt gat aaa tgt ttg 864
Arg Ala Lys Gly Thr Thr Gly Ser Glu Ala His Val Asp Lys Cys Leu
        275                 280                 285

gag ttg gca gag tat tta tac aac atc ata aaa aac cga gaa gga tat 912
Glu Leu Ala Glu Tyr Leu Tyr Asn Ile Ile Lys Asn Arg Glu Gly Tyr
    290                 295                 300

gag atg gtg ttt gat ggg aag cct cag cac aca aat gtc tgc ttc tgg 960
Glu Met Val Phe Asp Gly Lys Pro Gln His Thr Asn Val Cys Phe Trp
305                 310                 315                 320

tac att cct cca agc ttg cgt act ctg gaa gac aat gaa gag aga atg 1008
Tyr Ile Pro Pro Ser Leu Arg Thr Leu Glu Asp Asn Glu Glu Arg Met
                325                 330                 335

agt cgc ctc tcg aag gtg gct cca gtg att aaa gcc aga atg atg gag 1056
Ser Arg Leu Ser Lys Val Ala Pro Val Ile Lys Ala Arg Met Met Glu
            340                 345                 350

tat gga acc aca atg gtc agc tac caa ccc ttg gga gac aag gtc aat 1104
Tyr Gly Thr Thr Met Val Ser Tyr Gln Pro Leu Gly Asp Lys Val Asn
        355                 360                 365

ttc ttc cgc atg gtc atc tca aac cca gcg gca act cac caa gac att 1152
Phe Phe Arg Met Val Ile Ser Asn Pro Ala Ala Thr His Gln Asp Ile
    370                 375                 380

gac ttc ctg att gaa gaa ata gaa cgc ctt gga caa gat tta cac cac 1200
Asp Phe Leu Ile Glu Glu Ile Glu Arg Leu Gly Gln Asp Leu His His
385                 390                 395                 400

cac cac cac cac taa 1215
His His His His

<210> 10
<211> 404
<212> PRT
<213> human GAD65 1-187deletion mutant (F1)
<400> 10

Met Val Gly Leu Ala Ala Asp Trp Leu Thr Ser Thr Ala Asn Thr Asn
1               5                   10                  15

Met Phe Thr Tyr Glu Ile Ala Pro Val Phe Val Leu Leu Glu Tyr Val
            20                  25                  30

Thr Leu Lys Lys Met Arg Glu Ile Ile Gly Trp Pro Gly Gly Ser Gly
        35                  40                  45

Asp Gly Ile Phe Ser Pro Gly Gly Ala Ile Ser Asn Met Tyr Ala Met
    50                  55                  60

Met Ile Ala Arg Phe Lys Met Phe Pro Glu Val Lys Glu Lys Gly Met
65                  70                  75                  80

Ala Ala Leu Pro Arg Leu Ile Ala Phe Thr Ser Glu His Ser His Phe
                85                  90                  95

Ser Leu Lys Lys Gly Ala Ala Ala Leu Gly Ile Gly Thr Asp Ser Val
            100                 105                 110

Ile Leu Ile Lys Cys Asp Glu Arg Gly Lys Met Ile Pro Ser Asp Leu
        115                 120                 125

Glu Arg Arg Ile Leu Glu Ala Lys Gln Lys Gly Phe Val Pro Phe Leu
    130                 135                 140

Val Ser Ala Thr Ala Gly Thr Thr Val Tyr Gly Ala Phe Asp Pro Leu
145                 150                 155                 160

Leu Ala Val Ala Asp Ile Cys Lys Lys Tyr Lys Ile Trp Ile His Val
                165                 170                 175

Asp Ala Ala Trp Gly Gly Gly Leu Leu Met Ser Arg Lys His Lys Trp
            180                 185                 190

Lys Leu Ser Gly Val Glu Arg Ala Asn Ser Val Thr Trp Asn Pro His
        195                 200                 205

Lys Met Met Gly Val Pro Leu Gln Cys Ser Ala Leu Leu Val Arg Glu
    210                 215                 220

Glu Gly Leu Met Gln Asn Cys Asn Gln Met His Ala Ser Tyr Leu Phe
225                 230                 235                 240

Gln Gln Asp Lys His Tyr Asp Leu Ser Tyr Asp Thr Gly Asp Lys Ala
                245                 250                 255

Leu Gln Cys Gly Arg His Val Asp Val Phe Lys Leu Trp Leu Met Trp
            260                 265                 270

Arg Ala Lys Gly Thr Thr Gly Ser Glu Ala His Val Asp Lys Cys Leu
        275                 280                 285

Glu Leu Ala Glu Tyr Leu Tyr Asn Ile Ile Lys Asn Arg Glu Gly Tyr
    290                 295                 300

Glu Met Val Phe Asp Gly Lys Pro Gln His Thr Asn Val Cys Phe Trp
305                 310                 315                 320

Tyr Ile Pro Pro Ser Leu Arg Thr Leu Glu Asp Asn Glu Glu Arg Met
                325                 330                 335

Ser Arg Leu Ser Lys Val Ala Pro Val Ile Lys Ala Arg Met Met Glu
            340                 345                 350

Tyr Gly Thr Thr Met Val Ser Tyr Gln Pro Leu Gly Asp Lys Val Asn
        355                 360                 365

Phe Phe Arg Met Val Ile Ser Asn Pro Ala Ala Thr His Gln Asp Ile
    370                 375                 380

Asp Phe Leu Ile Glu Glu Ile Glu Arg Leu Gly Gln Asp Leu His His
385                 390                 395                 400

His His His His

<210>  11

<211>  1215

<212>  DNA

<213>  human GAD65 1-187deletion mutant(F86)

<220>

<221>  CDS

<222>  (1)..(1212)

<223>

<400>  11

atg gtt gga tta gca gca gac tgg ctg aca tca aca gca aat act aac        48

Met Val Gly Leu Ala Ala Asp Trp Leu Thr Ser Thr Ala Asn Thr Asn

1               5                   10                  15

atg ttc acc tat gaa att gct cca gta ttt gtg ctt ttg gaa tat gtc        96

Met Phe Thr Tyr Glu Ile Ala Pro Val Phe Val Leu Leu Glu Tyr Val

            20                  25                  30

aca cta aag aaa atg aga gaa atc att ggc tgg cca ggg ggc tct ggc        144

Thr Leu Lys Lys Met Arg Glu Ile Ile Gly Trp Pro Gly Gly Ser Gly

        35                  40                  45

gat ggg ata ttt tct ccc ggt ggc gcc ata tct aac atg tat gcc atg        192

Asp Gly Ile Phe Ser Pro Gly Gly Ala Ile Ser Asn Met Tyr Ala Met

    50                  55                  60

atg atc gca cgc ttt aag atg ttc cca gaa gtc aag gag aaa gga atg 240
Met Ile Ala Arg Phe Lys Met Phe Pro Glu Val Lys Glu Lys Gly Met
65                  70                  75                  80

gct gct ctt ccc agg ctc att gcc ttc acg tct gaa cat agt cat ttt 288
Ala Ala Leu Pro Arg Leu Ile Ala Phe Thr Ser Glu His Ser His Phe
                85                  90                  95

tct ctc aag aag gga gct gca gcc tta ggg att gga aca gac agc gtg 336
Ser Leu Lys Lys Gly Ala Ala Ala Leu Gly Ile Gly Thr Asp Ser Val
            100                 105                 110

att ctg att aaa tgt gat gag aga ggg aaa atg att cca tct gat ctt 384
Ile Leu Ile Lys Cys Asp Glu Arg Gly Lys Met Ile Pro Ser Asp Leu
        115                 120                 125

gaa aga agg att ctt gaa gcc aaa cag aaa ggg ttt gtt cct ttc ctc 432
Glu Arg Arg Ile Leu Glu Ala Lys Gln Lys Gly Phe Val Pro Phe Leu
    130                 135                 140

gtg agt gcc aca gct gga acc acc gtg tac gga gca ttt gac ccc ctc 480
Val Ser Ala Thr Ala Gly Thr Thr Val Tyr Gly Ala Phe Asp Pro Leu
145                 150                 155                 160

tta gct gtc gct gac att tgc aga aag tat aag atc tgg atg cat gtg 528
Leu Ala Val Ala Asp Ile Cys Arg Lys Tyr Lys Ile Trp Met His Val
                165                 170                 175

gat gca gct tgg ggt ggg gga tta ctg atg tcc cga aaa cac aag tgg 576
Asp Ala Ala Trp Gly Gly Gly Leu Leu Met Ser Arg Lys His Lys Trp
            180                 185                 190

aaa ctg agt ggc gtg gag agg gcc aac tct gtg acg tgg aat cca cac 624
Lys Leu Ser Gly Val Glu Arg Ala Asn Ser Val Thr Trp Asn Pro His
        195                 200                 205

aag atg atg gga gtc cct ttg cag tgc tct gct ctc ctg gtt aga gaa 672
Lys Met Met Gly Val Pro Leu Gln Cys Ser Ala Leu Leu Val Arg Glu
    210                 215                 220

gag gga ttg atg cag aat tgc aac caa atg cat gcc tcc tac ctc ttt 720
Glu Gly Leu Met Gln Asn Cys Asn Gln Met His Ala Ser Tyr Leu Phe
225                 230                 235                 240

cag caa gat aaa cat tat gac ctg tcc tat gac act gga gac aag gcc 768
Gln Gln Asp Lys His Tyr Asp Leu Ser Tyr Asp Thr Gly Asp Lys Ala
                245                 250                 255

tta cag tgc gga cgc cac gtt gat gtt ttt aaa cta tgg ctg atg tgg 816
Leu Gln Cys Gly Arg His Val Asp Val Phe Lys Leu Trp Leu Met Trp
            260                 265                 270

agg gca aag ggg act acc ggg ttt gaa gcg cat gtt gat aaa tgt ttg 864
Arg Ala Lys Gly Thr Thr Gly Phe Glu Ala His Val Asp Lys Cys Leu
        275                 280                 285

gag ttg gca gag tat tta tac aac atc ata aaa aac cga gaa gga tat 912

Glu Leu Ala Glu Tyr Leu Tyr Asn Ile Ile Lys Asn Arg Glu Gly Tyr
    290                 295                 300

gag atg gtg ttt gat ggg aag cct cag cac aca aat gtc tgc ttc tgg 960
Glu Met Val Phe Asp Gly Lys Pro Gln His Thr Asn Val Cys Phe Trp
305                 310                 315                 320

tac att cct cca agc ttg cgt act ctg gaa gac aat gaa gag aga atg 1008
Tyr Ile Pro Pro Ser Leu Arg Thr Leu Glu Asp Asn Glu Glu Arg Met
                325                 330                 335

agt cgc ctc tcg aag gtg gct cca gtg att aaa gcc aga atg atg gag 1056
Ser Arg Leu Ser Lys Val Ala Pro Val Ile Lys Ala Arg Met Met Glu
            340                 345                 350

tat gga acc aca atg gtc agc tac caa ccc ttg gga gac aag gtc aat 1104
Tyr Gly Thr Thr Met Val Ser Tyr Gln Pro Leu Gly Asp Lys Val Asn
        355                 360                 365

ttc ttc cgc atg gtc atc tca aac cca gcg gca act cac caa gac att 1152
Phe Phe Arg Met Val Ile Ser Asn Pro Ala Ala Thr His Gln Asp Ile
    370                 375                 380

gac ttc ctg att gaa gaa ata gaa cgc ctt gga caa gat tta cac cac 1200
Asp Phe Leu Ile Glu Glu Ile Glu Arg Leu Gly Gln Asp Leu His His
385                 390                 395                 400

cac cac cac cac taa 1215
His His His His

<210> 12

<211> 404

<212> PRT

<213> human GAD65 1-187deletion mutant(F86)

<400> 12

Met Val Gly Leu Ala Ala Asp Trp Leu Thr Ser Thr Ala Asn Thr Asn
1               5                   10                  15

Met Phe Thr Tyr Glu Ile Ala Pro Val Phe Val Leu Leu Glu Tyr Val
            20                  25                  30

Thr Leu Lys Lys Met Arg Glu Ile Ile Gly Trp Pro Gly Gly Ser Gly
        35                  40                  45

Asp Gly Ile Phe Ser Pro Gly Gly Ala Ile Ser Asn Met Tyr Ala Met
    50                  55                  60

Met Ile Ala Arg Phe Lys Met Phe Pro Glu Val Lys Glu Lys Gly Met

65                  70                  75                  80

Ala Ala Leu Pro Arg Leu Ile Ala Phe Thr Ser Glu His Ser His Phe
                85                  90                  95

Ser Leu Lys Lys Gly Ala Ala Ala Leu Gly Ile Gly Thr Asp Ser Val
            100                 105                 110

Ile Leu Ile Lys Cys Asp Glu Arg Gly Lys Met Ile Pro Ser Asp Leu
        115                 120                 125

Glu Arg Arg Ile Leu Glu Ala Lys Gln Lys Gly Phe Val Pro Phe Leu
    130                 135                 140

Val Ser Ala Thr Ala Gly Thr Thr Val Tyr Gly Ala Phe Asp Pro Leu
145                 150                 155                 160

Leu Ala Val Ala Asp Ile Cys Arg Lys Tyr Lys Ile Trp Met His Val
                165                 170                 175

Asp Ala Ala Trp Gly Gly Gly Leu Leu Met Ser Arg Lys His Lys Trp
            180                 185                 190

Lys Leu Ser Gly Val Glu Arg Ala Asn Ser Val Thr Trp Asn Pro His
        195                 200                 205

Lys Met Met Gly Val Pro Leu Gln Cys Ser Ala Leu Leu Val Arg Glu
    210                 215                 220

Glu Gly Leu Met Gln Asn Cys Asn Gln Met His Ala Ser Tyr Leu Phe
225                 230                 235                 240

Gln Gln Asp Lys His Tyr Asp Leu Ser Tyr Asp Thr Gly Asp Lys Ala
                245                 250                 255

Leu Gln Cys Gly Arg His Val Asp Val Phe Lys Leu Trp Leu Met Trp
            260                 265                 270

Arg Ala Lys Gly Thr Thr Gly Phe Glu Ala His Val Asp Lys Cys Leu
        275                 280                 285

Glu Leu Ala Glu Tyr Leu Tyr Asn Ile Ile Lys Asn Arg Glu Gly Tyr
    290                 295                 300

Glu Met Val Phe Asp Gly Lys Pro Gln His Thr Asn Val Cys Phe Trp
305                 310                 315                 320

Tyr Ile Pro Pro Ser Leu Arg Thr Leu Glu Asp Asn Glu Glu Arg Met
                325                 330                 335

Ser Arg Leu Ser Lys Val Ala Pro Val Ile Lys Ala Arg Met Met Glu
            340                 345                 350

Tyr Gly Thr Thr Met Val Ser Tyr Gln Pro Leu Gly Asp Lys Val Asn
        355                 360                 365

Phe Phe Arg Met Val Ile Ser Asn Pro Ala Ala Thr His Gln Asp Ile
    370                 375                 380

Asp Phe Leu Ile Glu Glu Ile Glu Arg Leu Gly Gln Asp Leu His His
385                 390                 395                 400

His His His His

<210> 13

<211> 1215

<212> DNA

<213> human GAD65 1-187deletion mutant(F87)

<220>

<221> CDS

<222> (1)..(1212)

<223>

<400> 13

atg gtt gga tta gca gca gac tgg ctg aca tca aca gca aat act aac 48

Met Val Gly Leu Ala Ala Asp Trp Leu Thr Ser Thr Ala Asn Thr Asn

1               5                   10                  15

atg ttc acc tat gaa att gct cca gta ttt gtg ctt ttg gaa tat gtc 96

Met Phe Thr Tyr Glu Ile Ala Pro Val Phe Val Leu Leu Glu Tyr Val

            20                  25                  30

aca cta aag aaa atg aga gaa atc att ggc tgg cca ggg ggc tct ggc 144

Thr Leu Lys Lys Met Arg Glu Ile Ile Gly Trp Pro Gly Gly Ser Gly

        35                  40                  45

gat ggg ata ttt tct ccc ggt ggc gcc ata tct aac atg tat gcc atg 192

Asp Gly Ile Phe Ser Pro Gly Gly Ala Ile Ser Asn Met Tyr Ala Met

    50                  55                  60

atg atc gca cgc ttt aag atg ttc cca gaa gtc aag gag aaa gga atg 240

Met Ile Ala Arg Phe Lys Met Phe Pro Glu Val Lys Glu Lys Gly Met

65                  70                  75                  80

gct gct ctt ccc agg ctc att gcc ttc acg tct gaa cat agt cat ttt 288
Ala Ala Leu Pro Arg Leu Ile Ala Phe Thr Ser Glu His Ser His Phe
                85                  90                  95

tct ctc aag aag gga gct gca gcc tta ggg att gga aca gac agc gtg 336
Ser Leu Lys Lys Gly Ala Ala Ala Leu Gly Ile Gly Thr Asp Ser Val
            100                 105                 110

att ctg att aaa tgt gat gag aga ggg aaa atg att cca tct gat ctt 384
Ile Leu Ile Lys Cys Asp Glu Arg Gly Lys Met Ile Pro Ser Asp Leu
        115                 120                 125

gaa aga agg att ctt gaa gcc aaa cag aaa ggg ttt gtt cct ttc ctc 432
Glu Arg Arg Ile Leu Glu Ala Lys Gln Lys Gly Phe Val Pro Phe Leu
    130                 135                 140

gtg agt gcc aca gct gga acc acc gtg tac gga gca ttt gtc ccc ctc 480
Val Ser Ala Thr Ala Gly Thr Thr Val Tyr Gly Ala Phe Val Pro Leu
145                 150                 155                 160

tta gct gtc gct gac att tgc aaa aag tat aag atc tgg atg cat gtg 528
Leu Ala Val Ala Asp Ile Cys Lys Lys Tyr Lys Ile Trp Met His Val
                165                 170                 175

gat gca gct tgg ggt ggg gga tta ctg atg tcc cga aaa cac aag tgg 576
Asp Ala Ala Trp Gly Gly Gly Leu Leu Met Ser Arg Lys His Lys Trp
            180                 185                 190

aaa ctg agt ggc gtg gag agg gcc aac tct gtg acg tgg aat cca cac 624
Lys Leu Ser Gly Val Glu Arg Ala Asn Ser Val Thr Trp Asn Pro His
        195                 200                 205

aag atg atg gga gtc cct ttg cag tgc tct gct ctc ctg gtt aga gaa 672
Lys Met Met Gly Val Pro Leu Gln Cys Ser Ala Leu Leu Val Arg Glu
    210                 215                 220

gag gga ttg atg cag aat tgc aac caa atg cat gcc tcc tac ctc ttt 720
Glu Gly Leu Met Gln Asn Cys Asn Gln Met His Ala Ser Tyr Leu Phe
225                 230                 235                 240

cag caa gat aaa cat tat gac ctg tcc tat gac act gga gac aag gcc 768
Gln Gln Asp Lys His Tyr Asp Leu Ser Tyr Asp Thr Gly Asp Lys Ala
                245                 250                 255

tta cag tgc gga cgc cac gtt gat gtt ttt aaa cta tgg ctg atg tgg 816
Leu Gln Cys Gly Arg His Val Asp Val Phe Lys Leu Trp Leu Met Trp
            260                 265                 270

agg gca aag ggg act acc ggg ttt gaa gcg cat gtt gat aaa tgt ttg 864
Arg Ala Lys Gly Thr Thr Gly Phe Glu Ala His Val Asp Lys Cys Leu
        275                 280                 285

gag ttg gca gag tat tta tac aac atc ata aaa aac cga gaa gga tat 912
Glu Leu Ala Glu Tyr Leu Tyr Asn Ile Ile Lys Asn Arg Glu Gly Tyr
    290                 295                 300

gag atg gtg ttt gat ggg aag cct cag cac aca aat gtc tgc ttc tgg 960
Glu Met Val Phe Asp Gly Lys Pro Gln His Thr Asn Val Cys Phe Trp
305                 310                 315                 320

tac att cct cca agc ttg cgt act ctg gaa gac aat gaa gag aga atg 1008
Tyr Ile Pro Pro Ser Leu Arg Thr Leu Glu Asp Asn Glu Glu Arg Met
                325                 330                 335

agt cgc ctc tcg aag gtg gct cca gtg att aaa gcc aga atg atg gag 1056
Ser Arg Leu Ser Lys Val Ala Pro Val Ile Lys Ala Arg Met Met Glu
            340                 345                 350

tat gga acc aca atg gtc agc tac caa ccc ttg gga gac aag gtc aat 1104
Tyr Gly Thr Thr Met Val Ser Tyr Gln Pro Leu Gly Asp Lys Val Asn
        355                 360                 365

ttc ttc cgc atg gtc atc tca aac cca gcg gca act cac caa gac att 1152
Phe Phe Arg Met Val Ile Ser Asn Pro Ala Ala Thr His Gln Asp Ile
    370                 375                 380

gac ttc ctg att gaa gaa ata gaa cgc ctt gga caa gat tta cac cac 1200
Asp Phe Leu Ile Glu Glu Ile Glu Arg Leu Gly Gln Asp Leu His His
385                 390                 395                 400

cac cac cac cac taa 1215
His His His His

<210> 14

<211> 404

<212> PRT

<213> human GAD65 1-187deletion mutant(F87)

<400> 14

Met Val Gly Leu Ala Ala Asp Trp Leu Thr Ser Thr Ala Asn Thr Asn
1               5                   10                  15

Met Phe Thr Tyr Glu Ile Ala Pro Val Phe Val Leu Leu Glu Tyr Val
            20                  25                  30

Thr Leu Lys Lys Met Arg Glu Ile Ile Gly Trp Pro Gly Gly Ser Gly
        35                  40                  45

Asp Gly Ile Phe Ser Pro Gly Gly Ala Ile Ser Asn Met Tyr Ala Met
    50                  55                  60

Met Ile Ala Arg Phe Lys Met Phe Pro Glu Val Lys Glu Lys Gly Met
65                  70                  75                  80

Ala Ala Leu Pro Arg Leu Ile Ala Phe Thr Ser Glu His Ser His Phe
                85                  90                  95

Ser Leu Lys Lys Gly Ala Ala Ala Leu Gly Ile Gly Thr Asp Ser Val
            100                 105                 110

Ile Leu Ile Lys Cys Asp Glu Arg Gly Lys Met Ile Pro Ser Asp Leu
        115                 120                 125

Glu Arg Arg Ile Leu Glu Ala Lys Gln Lys Gly Phe Val Pro Phe Leu
    130                 135                 140

Val Ser Ala Thr Ala Gly Thr Thr Val Tyr Gly Ala Phe Val Pro Leu
145                 150                 155                 160

Leu Ala Val Ala Asp Ile Cys Lys Lys Tyr Lys Ile Trp Met His Val
                165                 170                 175

Asp Ala Ala Trp Gly Gly Gly Leu Leu Met Ser Arg Lys His Lys Trp
            180                 185                 190

Lys Leu Ser Gly Val Glu Arg Ala Asn Ser Val Thr Trp Asn Pro His
        195                 200                 205

Lys Met Met Gly Val Pro Leu Gln Cys Ser Ala Leu Leu Val Arg Glu
    210                 215                 220

Glu Gly Leu Met Gln Asn Cys Asn Gln Met His Ala Ser Tyr Leu Phe
225                 230                 235                 240

Gln Gln Asp Lys His Tyr Asp Leu Ser Tyr Asp Thr Gly Asp Lys Ala
                245                 250                 255

Leu Gln Cys Gly Arg His Val Asp Val Phe Lys Leu Trp Leu Met Trp
            260                 265                 270

Arg Ala Lys Gly Thr Thr Gly Phe Glu Ala His Val Asp Lys Cys Leu
        275                 280                 285

Glu Leu Ala Glu Tyr Leu Tyr Asn Ile Ile Lys Asn Arg Glu Gly Tyr
    290                 295                 300

Glu Met Val Phe Asp Gly Lys Pro Gln His Thr Asn Val Cys Phe Trp
305                 310                 315                 320

Tyr Ile Pro Pro Ser Leu Arg Thr Leu Glu Asp Asn Glu Glu Arg Met
                325                 330                 335

Ser Arg Leu Ser Lys Val Ala Pro Val Ile Lys Ala Arg Met Met Glu
            340                 345                 350

Tyr Gly Thr Thr Met Val Ser Tyr Gln Pro Leu Gly Asp Lys Val Asn
        355                 360                 365

Phe Phe Arg Met Val Ile Ser Asn Pro Ala Ala Thr His Gln Asp Ile
    370                 375                 380

Asp Phe Leu Ile Glu Glu Ile Glu Arg Leu Gly Gln Asp Leu His His
385                 390                 395                 400

His His His His

<210> 15
<211> 1215
<212> DNA
<213> human GAD65 1-187deletion mutant(F84)

<220>

<221> CDS

<222> (1)..(1212)

<223>

<400> 15

atg gtt gga tta gca gca gac tgg ctg aca tca aca gca aat act aac 48
Met Val Gly Leu Ala Ala Asp Trp Leu Thr Ser Thr Ala Asn Thr Asn
1               5                   10                  15

atg ttc acc tat gaa att gct cca gta ttt gtg ctt ttg gaa tat gtc 96
Met Phe Thr Tyr Glu Ile Ala Pro Val Phe Val Leu Leu Glu Tyr Val
            20                  25                  30

aca cta aag aaa atg aga gaa atc att ggc tgg cca ggg ggc tct ggc 144
Thr Leu Lys Lys Met Arg Glu Ile Ile Gly Trp Pro Gly Gly Ser Gly
        35                  40                  45

gat ggg ata ttt tct ccc ggt ggc gcc ata tct aac atg tat gcc atg 192
Asp Gly Ile Phe Ser Pro Gly Gly Ala Ile Ser Asn Met Tyr Ala Met
    50                  55                  60

atg atc gca cgc ttt aag atg ttc cca gaa gtc aag gag aaa gga atg 240
Met Ile Ala Arg Phe Lys Met Phe Pro Glu Val Lys Glu Lys Gly Met
65                  70                  75                  80

gct gct ctt ccc agg ctc att gcc ttc acg tct gaa cat agt cat ttt 288
Ala Ala Leu Pro Arg Leu Ile Ala Phe Thr Ser Glu His Ser His Phe
                85                  90                  95

tct ctc aag aag gga gct gca gcc tta ggg att gga aca gac agc gtg 336
Ser Leu Lys Lys Gly Ala Ala Ala Leu Gly Ile Gly Thr Asp Ser Val
            100                 105                 110

att ctg att aaa tgt gat gag aga ggg aaa atg att cca tct gat ctt 384
Ile Leu Ile Lys Cys Asp Glu Arg Gly Lys Met Ile Pro Ser Asp Leu
        115                 120                 125

gaa aga agg att ctt gaa gcc aaa cag aaa ggg ttt gtt cct ttc ctc 432
Glu Arg Arg Ile Leu Glu Ala Lys Gln Lys Gly Phe Val Pro Phe Leu
    130                 135                 140

gtg agt gcc aca gct gga acc acc gtg tac gga gca ttt gac ccc ctc 480
Val Ser Ala Thr Ala Gly Thr Thr Val Tyr Gly Ala Phe Asp Pro Leu
145                 150                 155                 160

tta gct gtc gct gac act tgc aaa aag tat aag atc tgg atg cat gtg 528
Leu Ala Val Ala Asp Thr Cys Lys Lys Tyr Lys Ile Trp Met His Val
                165                 170                 175

gat gca gct tgg ggt ggg gga tta ctg atg tcc cga aaa cac aag tgg 576
Asp Ala Ala Trp Gly Gly Gly Leu Leu Met Ser Arg Lys His Lys Trp
            180                 185                 190

aaa ctg agt ggc gtg gag agg gcc aac tct gtg acg tgg aat cca cac 624
Lys Leu Ser Gly Val Glu Arg Ala Asn Ser Val Thr Trp Asn Pro His
        195                 200                 205

aag atg atg gga gtc cct ttg cag tgc tct gct ctc ctg gtt aga gaa 672
Lys Met Met Gly Val Pro Leu Gln Cys Ser Ala Leu Leu Val Arg Glu
    210                 215                 220

gag gga ttg atg cag aat tgc aac caa atg cat gcc tcc tac ctc ttt 720
Glu Gly Leu Met Gln Asn Cys Asn Gln Met His Ala Ser Tyr Leu Phe
225                 230                 235                 240

cag caa gat aaa cat tat gac ctg tcc tat gac act gga gac aag gcc 768
Gln Gln Asp Lys His Tyr Asp Leu Ser Tyr Asp Thr Gly Asp Lys Ala
                245                 250                 255

tta cag tgc gga cgc cac gtt gat gtt ttt aaa cta tgg ctg atg tgg 816
Leu Gln Cys Gly Arg His Val Asp Val Phe Lys Leu Trp Leu Met Trp
            260                 265                 270

agg gca aag ggg act acc ggg ttt gaa gcg cat gtt gat aaa tgt ttg 864
Arg Ala Lys Gly Thr Thr Gly Phe Glu Ala His Val Asp Lys Cys Leu
        275                 280                 285

gag ttg gca gag tat tta tac aac atc ata aaa aac cga gaa gga tat 912
Glu Leu Ala Glu Tyr Leu Tyr Asn Ile Ile Lys Asn Arg Glu Gly Tyr
    290                 295                 300

gag atg gtg ttt gat ggg aag cct cag cac aca aat gtc tgc ttc tgg 960
Glu Met Val Phe Asp Gly Lys Pro Gln His Thr Asn Val Cys Phe Trp
305                 310                 315                 320

tac att cct cca agc ttg cgt act ctg gaa gac aat gaa gag aga atg 1008

Tyr Ile Pro Pro Ser Leu Arg Thr Leu Glu Asp Asn Glu Glu Arg Met
                325                 330                 335

agt cgc ctc tcg aag gtg gct cca gtg att aaa gcc aga atg atg gag 1056
Ser Arg Leu Ser Lys Val Ala Pro Val Ile Lys Ala Arg Met Met Glu
            340                 345                 350

tat gga acc aca atg gtc agc tac caa ccc ttg gga gac aag gtc aat 1104
Tyr Gly Thr Thr Met Val Ser Tyr Gln Pro Leu Gly Asp Lys Val Asn
        355                 360                 365

ttc ttc cgc atg gtc atc tca aac cca gcg gca act cac caa gac att 1152
Phe Phe Arg Met Val Ile Ser Asn Pro Ala Ala Thr His Gln Asp Ile
    370                 375                 380

gac ttc ctg att gaa gaa ata gaa cgc ctt gga caa gat tta cac cac 1200
Asp Phe Leu Ile Glu Glu Ile Glu Arg Leu Gly Gln Asp Leu His His
385                 390                 395                 400

cac cac cac cac taa 1215
His His His His

<210> 16
<211> 404
<212> PRT
<213> human GAD65 1-187deletion mutant(F84)

<400> 16

Met Val Gly Leu Ala Ala Asp Trp Leu Thr Ser Thr Ala Asn Thr Asn
1               5                   10                  15

Met Phe Thr Tyr Glu Ile Ala Pro Val Phe Val Leu Leu Glu Tyr Val
            20                  25                  30

Thr Leu Lys Lys Met Arg Glu Ile Ile Gly Trp Pro Gly Gly Ser Gly
        35                  40                  45

Asp Gly Ile Phe Ser Pro Gly Gly Ala Ile Ser Asn Met Tyr Ala Met
    50                  55                  60

Met Ile Ala Arg Phe Lys Met Phe Pro Glu Val Lys Glu Lys Gly Met
65                  70                  75                  80

Ala Ala Leu Pro Arg Leu Ile Ala Phe Thr Ser Glu His Ser His Phe
                85                  90                  95

Ser Leu Lys Lys Gly Ala Ala Ala Leu Gly Ile Gly Thr Asp Ser Val
            100                 105                 110

Ile Leu Ile Lys Cys Asp Glu Arg Gly Lys Met Ile Pro Thr Asp Leu
        115                 120                 125

Glu Arg Arg Ile Leu Glu Ala Lys Gln Lys Gly Phe Val Pro Phe Leu
    130                 135                 140

Val Ser Ala Thr Ala Gly Thr Thr Val Tyr Gly Ala Phe Asp Pro Leu
145                 150                 155                 160

Leu Ala Val Ala Asp Thr Cys Lys Lys Tyr Lys Ile Trp Met His Val
                165                 170                 175

Asp Ala Ala Trp Gly Gly Gly Leu Leu Met Ser Arg Lys His Lys Trp
            180                 185                 190

Lys Leu Ser Gly Val Glu Arg Ala Asn Ser Val Thr Trp Asn Pro His
        195                 200                 205

Lys Met Met Gly Val Pro Leu Gln Cys Ser Ala Leu Leu Val Arg Glu
    210                 215                 220

Glu Gly Leu Met Gln Asn Cys Asn Gln Met His Ala Ser Tyr Leu Phe
225                 230                 235                 240

Gln Gln Asp Lys His Tyr Asp Leu Ser Tyr Asp Thr Gly Asp Lys Ala
                245                 250                 255

Leu Gln Cys Gly Arg His Val Asp Val Phe Lys Leu Trp Leu Met Trp
            260                 265                 270

Arg Ala Lys Gly Thr Thr Gly Phe Glu Ala His Val Asp Lys Cys Leu
        275                 280                 285

Glu Leu Ala Glu Tyr Leu Tyr Asn Ile Ile Lys Asn Arg Glu Gly Tyr
    290                 295                 300

Glu Met Val Phe Asp Gly Lys Pro Gln His Thr Asn Val Cys Phe Trp
305                 310                 315                 320

Tyr Ile Pro Pro Ser Leu Arg Thr Leu Glu Asp Asn Glu Glu Arg Met
                325                 330                 335

Ser Arg Leu Ser Lys Val Ala Pro Val Ile Lys Ala Arg Met Met Glu

            340                 345                 350

Tyr Gly Thr Thr Met Val Ser Tyr Gln Pro Leu Gly Asp Lys Val Asn
        355                 360                 365

Phe Phe Arg Met Val Ile Ser Asn Pro Ala Ala Thr His Gln Asp Ile
    370                 375                 380

Asp Phe Leu Ile Glu Glu Ile Glu Arg Leu Gly Gln Asp Leu His His
385                 390                 395                 400

His His His His

<210> 17
<211> 36
<212> DNA
<213> Artificial

<220>
<223> primer

<400> 17
ttgaattcac catggttgga ttagcagcag actggc 36

<210> 18
<211> 23
<212> DNA
<213> Artificial

<220>
<223> primer

<400> 18
ggcaaatggc attctgacat cct 23

<210> 19
<211> 18
<212> DNA
<213> Artificial

<220>
<223> primer

<400> 19
ttcctcgacg ctaacctg 18

<210> 20
<211> 20
<212> DNA

<213> Artificial

<220>
<223> primer

<400> 20
tgtaaaacga cggccagtgc 20

<210> 21
<211> 33
<212> DNA
<213> Artificial

<220>
<223> primer

<400> 21
ttgaattcac catggttgga ttagcagact ggc 33

<210> 22
<211> 31
<212> DNA
<213> Artificial

<220>
<223> primer

<400> 22
tcctggttag agaagcggga ttgatgcaga a 31

<210> 23
<211> 33
<212> DNA
<213> Artificial

<220>
<223> primer

<400> 23
tagcggccgc ttagtggtgg tggtggtggt gag 33

<210> 24
<211> 31
<212> DNA
<213> Artificial

<220>
<223> primer

<400> 24
ttctgcatca atcccgcttc tctaaccagg a 31

<210> 25

<211>  42

<212>  DNA

<213>  Artificial

<220>

<223>  primer

<400>  25

tagaattcac catggtgctg cgggtgtgat ggagaaaggc cc                                42

<210>  26

<211>  23

<212>  DNA

<213>  Artificial

<220>

<223>  primer

<400>  26

ggcaaatggc attctgacat cct

<210>  27

<211>  342

<212>  DNA

<213>  E.coli(Thioredoxin partial sequence)

<220>

<221> CDS

<222> (1)..(342)

<223>

<400> 27

atg tct gat aaa att att cat ctg act gat gat tct ttt gat act gat 48

Met Ser Asp Lys Ile Ile His Leu Thr Asp Asp Ser Phe Asp Thr Asp

1               5                   10                  15

gta ctt aag gca gat ggt gca atc ctg gtt gat ttc tgg gca cac tgg 96

Val Leu Lys Ala Asp Gly Ala Ile Leu Val Asp Phe Trp Ala His Trp

            20                  25                  30

tgc ggt ccg tgc aaa atg atc gct ccg att ctg gat gaa atc gct gac 144

Cys Gly Pro Cys Lys Met Ile Ala Pro Ile Leu Asp Glu Ile Ala Asp

        35                  40                  45

gaa tat cag ggc aaa ctg acc gtt gca aaa ctg aac atc gat cac aac 192

Glu Tyr Gln Gly Lys Leu Thr Val Ala Lys Leu Asn Ile Asp His Asn

    50                  55                  60

ccg ggc act gcg ccg aaa tat ggc atc cgt ggt atc ccg act ctg ctg 240

Pro Gly Thr Ala Pro Lys Tyr Gly Ile Arg Gly Ile Pro Thr Leu Leu

65                  70                  75                  80

ctg ttc aaa aac ggt gaa gtg gcg gca acc aaa gtg ggt gca ctg tct 288

Leu Phe Lys Asn Gly Glu Val Ala Ala Thr Lys Val Gly Ala Leu Ser

                85                  90                  95

aaa ggt cag ttg aaa gag ttc ctc gac gct aac ctg gcc ggc tct gga 336
Lys Gly Gln Leu Lys Glu Phe Leu Asp Ala Asn Leu Ala Gly Ser Gly
            100                 105                 110

tcc ggt 342
Ser Gly

<210> 28
<211> 114
<212> PRT
<213> E.coli(Thioredoxin partial sequence)

<400> 28

Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala His Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp His Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly

<210>  29
<211>  536
<212>  DNA
<213>  Artificial

<220>
<223>  pThioHisA multiple cloning site

<220>

<221> CDS

<222> (3)..(437)

<223>

<400> 29

at atg tct gat aaa att att cat ctg act gat gat tct ttt gat act 47

Met Ser Asp Lys Ile Ile His Leu Thr Asp Asp Ser Phe Asp Thr

1               5                   10                  15

gat gta ctt aag gca gat ggt gca atc ctg gtt gat ttc tgg gca cac 95

Asp Val Leu Lys Ala Asp Gly Ala Ile Leu Val Asp Phe Trp Ala His

                20                  25                  30

tgg tgc ggt ccg tgc aaa atg atc gct ccg att ctg gat gaa atc gct 143

Trp Cys Gly Pro Cys Lys Met Ile Ala Pro Ile Leu Asp Glu Ile Ala

            35                  40                  45

gac gaa tat cag ggc aaa ctg acc gtt gca aaa ctg aac atc gat cac 191

Asp Glu Tyr Gln Gly Lys Leu Thr Val Ala Lys Leu Asn Ile Asp His

        50                  55                  60

aac ccg ggc act gcg ccg aaa tat ggc atc cgt ggt atc ccg act ctg 239

Asn Pro Gly Thr Ala Pro Lys Tyr Gly Ile Arg Gly Ile Pro Thr Leu

    65                  70                  75

ctg ctg ttc aaa aac ggt gaa gtg gcg gca acc aaa gtg ggt gca ctg 287

Leu Leu Phe Lys Asn Gly Glu Val Ala Ala Thr Lys Val Gly Ala Leu

80                  85                  90                  95

tct aaa ggt cag ttg aaa gag ttc ctc gac gct aac ctg gcc ggc tct     335
Ser Lys Gly Gln Leu Lys Glu Phe Leu Asp Ala Asn Leu Ala Gly Ser
                100                 105                 110

gga tcc ggt gat gac gat gac aag gta cct atg cat gag ctc gag atc     383
Gly Ser Gly Asp Asp Asp Asp Lys Val Pro Met His Glu Leu Glu Ile
            115                 120                 125

ttc gaa ttc cgc ggc cgc agg cct cta gag tcg acc tgc agt aat cgt     431
Phe Glu Phe Arg Gly Arg Arg Pro Leu Glu Ser Thr Cys Ser Asn Arg
        130                 135                 140

aca ggg tagtacaaat aaaaaaggca cgtcagatga cgtgcctttt ttcttgtgag     487
Thr Gly
    145

cagtaagctt ggcactggcc gtcgttttac aacgtcgtga ctgggaaaa     536

<210>  30
<211>  145
<212>  PRT
<213>  Artificial

<220>
<223>  pThioHisA multiple cloning site

<400>  30

Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala His Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp His Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Ala Thr Lys Val Gly Ala Leu Ser
                85                  90                  95

Lys Gly Gln Leu Lys Glu Phe Leu Asp Ala Asn Leu Ala Gly Ser Gly
            100                 105                 110

Ser Gly Asp Asp Asp Asp Lys Val Pro Met His Glu Leu Glu Ile Phe
        115                 120                 125

Glu Phe Arg Gly Arg Arg Pro Leu Glu Ser Thr Cys Ser Asn Arg Thr
    130                 135                 140

Gly
145

<210>  31
<211>  1215
<212>  DNA
<213>  human GAD65 1-187deletion (wild type)

<220>
<221>  CDS
<222>  (1)..(1212)
<223>

<400>  31

atg gtt gga tta gca gca gac tgg ctg aca tca aca gca aat act aac      48
Met Val Gly Leu Ala Ala Asp Trp Leu Thr Ser Thr Ala Asn Thr Asn
1               5                   10                  15

atg ttc acc tat gaa att gct cca gta ttt gtg ctt ttg gaa tat gtc      96

Met Phe Thr Tyr Glu Ile Ala Pro Val Phe Val Leu Leu Glu Tyr Val
            20                  25                  30

aca cta aag aaa atg aga gaa atc att ggc tgg cca ggg ggc tct ggc 144
Thr Leu Lys Lys Met Arg Glu Ile Ile Gly Trp Pro Gly Gly Ser Gly
        35                  40                  45

gat ggg ata ttt tct ccc ggt ggc gcc ata tct aac atg tat gcc atg 192
Asp Gly Ile Phe Ser Pro Gly Gly Ala Ile Ser Asn Met Tyr Ala Met
    50                  55                  60

atg atc gca cgc ttt aag atg ttc cca gaa gtc aag gag aaa gga atg 240
Met Ile Ala Arg Phe Lys Met Phe Pro Glu Val Lys Glu Lys Gly Met
65                  70                  75                  80

gct gct ctt ccc agg ctc att gcc ttc acg tct gaa cat agt cat ttt 288
Ala Ala Leu Pro Arg Leu Ile Ala Phe Thr Ser Glu His Ser His Phe
                85                  90                  95

tct ctc aag aag gga gct gca gcc tta ggg att gga aca gac agc gtg 336
Ser Leu Lys Lys Gly Ala Ala Ala Leu Gly Ile Gly Thr Asp Ser Val
            100                 105                 110

att ctg att aaa tgt gat gag aga ggg aaa atg att cca tct gat ctt 384
Ile Leu Ile Lys Cys Asp Glu Arg Gly Lys Met Ile Pro Ser Asp Leu
        115                 120                 125

gaa aga agg att ctt gaa gcc aaa cag aaa ggg ttt gtt cct ttc ctc 432
Glu Arg Arg Ile Leu Glu Ala Lys Gln Lys Gly Phe Val Pro Phe Leu

    130                 135                 140

gtg agt gcc aca gct gga acc acc gtg tac gga gca ttt gac ccc ctc 480
Val Ser Ala Thr Ala Gly Thr Thr Val Tyr Gly Ala Phe Asp Pro Leu
145                 150                 155                 160

tta gct gtc gct gac att tgc aaa aag tat aag atc tgg atg cat gtg 528
Leu Ala Val Ala Asp Ile Cys Lys Lys Tyr Lys Ile Trp Met His Val
                165                 170                 175

gat gca gct tgg ggt ggg gga tta ctg atg tcc cga aaa cac aag tgg 576
Asp Ala Ala Trp Gly Gly Gly Leu Leu Met Ser Arg Lys His Lys Trp
            180                 185                 190

aaa ctg agt ggc gtg gag agg gcc aac tct gtg acg tgg aat cca cac 624
Lys Leu Ser Gly Val Glu Arg Ala Asn Ser Val Thr Trp Asn Pro His
        195                 200                 205

aag atg atg gga gtc cct ttg cag tgc tct gct ctc ctg gtt aga gaa 672
Lys Met Met Gly Val Pro Leu Gln Cys Ser Ala Leu Leu Val Arg Glu
    210                 215                 220

gag gga ttg atg cag aat tgc aac caa atg cat gcc tcc tac ctc ttt 720
Glu Gly Leu Met Gln Asn Cys Asn Gln Met His Ala Ser Tyr Leu Phe
225                 230                 235                 240

cag caa gat aaa cat tat gac ctg tcc tat gac act gga gac aag gcc 768
Gln Gln Asp Lys His Tyr Asp Leu Ser Tyr Asp Thr Gly Asp Lys Ala
                245                 250                 255

tta cag tgc gga cgc cac gtt gat gtt ttt aaa cta tgg ctg atg tgg 816
Leu Gln Cys Gly Arg His Val Asp Val Phe Lys Leu Trp Leu Met Trp
            260                 265                 270

agg gca aag ggg act acc ggg ttt gaa gcg cat gtt gat aaa tgt ttg 864
Arg Ala Lys Gly Thr Thr Gly Phe Glu Ala His Val Asp Lys Cys Leu
        275                 280                 285

gag ttg gca gag tat tta tac aac atc ata aaa aac cga gaa gga tat 912
Glu Leu Ala Glu Tyr Leu Tyr Asn Ile Ile Lys Asn Arg Glu Gly Tyr
    290                 295                 300

gag atg gtg ttt gat ggg aag cct cag cac aca aat gtc tgc ttc tgg 960
Glu Met Val Phe Asp Gly Lys Pro Gln His Thr Asn Val Cys Phe Trp
305                 310                 315                 320

tac att cct cca agc ttg cgt act ctg gaa gac aat gaa gag aga atg 1008
Tyr Ile Pro Pro Ser Leu Arg Thr Leu Glu Asp Asn Glu Glu Arg Met
                325                 330                 335

agt cgc ctc tcg aag gtg gct cca gtg att aaa gcc aga atg atg gag 1056
Ser Arg Leu Ser Lys Val Ala Pro Val Ile Lys Ala Arg Met Met Glu
            340                 345                 350

tat gga acc aca atg gtc agc tac caa ccc ttg gga gac aag gtc aat 1104
Tyr Gly Thr Thr Met Val Ser Tyr Gln Pro Leu Gly Asp Lys Val Asn
        355                 360                 365

ttc ttc cgc atg gtc atc tca aac cca gcg gca act cac caa gac att 1152
Phe Phe Arg Met Val Ile Ser Asn Pro Ala Ala Thr His Gln Asp Ile
    370                 375                 380

gac ttc ctg att gaa gaa ata gaa cgc ctt gga caa gat tta cac cac 1200
Asp Phe Leu Ile Glu Glu Ile Glu Arg Leu Gly Gln Asp Leu His His
385                 390                 395                 400

cac cac cac cac taa 1215
His His His His

<210> 32
<211> 404
<212> PRT
<213> human GAD65 1-187deletion (wild type)

<400> 32

Met Val Gly Leu Ala Ala Asp Trp Leu Thr Ser Thr Ala Asn Thr Asn
1               5                   10                  15

Met Phe Thr Tyr Glu Ile Ala Pro Val Phe Val Leu Leu Glu Tyr Val
            20                  25                  30

Thr Leu Lys Lys Met Arg Glu Ile Ile Gly Trp Pro Gly Gly Ser Gly

        35                  40                  45

Asp Gly Ile Phe Ser Pro Gly Gly Ala Ile Ser Asn Met Tyr Ala Met
    50                  55                  60

Met Ile Ala Arg Phe Lys Met Phe Pro Glu Val Lys Glu Lys Gly Met
65                  70                  75                  80

Ala Ala Leu Pro Arg Leu Ile Ala Phe Thr Ser Glu His Ser His Phe
                85                  90                  95

Ser Leu Lys Lys Gly Ala Ala Ala Leu Gly Ile Gly Thr Asp Ser Val
            100                 105                 110

Ile Leu Ile Lys Cys Asp Glu Arg Gly Lys Met Ile Pro Ser Asp Leu
        115                 120                 125

Glu Arg Arg Ile Leu Glu Ala Lys Gln Lys Gly Phe Val Pro Phe Leu
    130                 135                 140

Val Ser Ala Thr Ala Gly Thr Thr Val Tyr Gly Ala Phe Asp Pro Leu
145                 150                 155                 160

Leu Ala Val Ala Asp Ile Cys Lys Lys Tyr Lys Ile Trp Met His Val
                165                 170                 175
Asp Ala Ala Trp Gly Gly Gly Leu Leu Met Ser Arg Lys His Lys Trp
            180                 185                 190
Lys Leu Ser Gly Val Glu Arg Ala Asn Ser Val Thr Trp Asn Pro His
        195                 200                 205
Lys Met Met Gly Val Pro Leu Gln Cys Ser Ala Leu Leu Val Arg Glu
    210                 215                 220
Glu Gly Leu Met Gln Asn Cys Asn Gln Met His Ala Ser Tyr Leu Phe
225                 230                 235                 240
Gln Gln Asp Lys His Tyr Asp Leu Ser Tyr Asp Thr Gly Asp Lys Ala
                245                 250                 255
Leu Gln Cys Gly Arg His Val Asp Val Phe Lys Leu Trp Leu Met Trp
            260                 265                 270

Arg Ala Lys Gly Thr Thr Gly Phe Glu Ala His Val Asp Lys Cys Leu
        275                 280                 285

Glu Leu Ala Glu Tyr Leu Tyr Asn Ile Ile Lys Asn Arg Glu Gly Tyr
    290                 295                 300

Glu Met Val Phe Asp Gly Lys Pro Gln His Thr Asn Val Cys Phe Trp
305                 310                 315                 320

Tyr Ile Pro Pro Ser Leu Arg Thr Leu Glu Asp Asn Glu Glu Arg Met
                325                 330                 335

Ser Arg Leu Ser Lys Val Ala Pro Val Ile Lys Ala Arg Met Met Glu
            340                 345                 350

Tyr Gly Thr Thr Met Val Ser Tyr Gln Pro Leu Gly Asp Lys Val Asn
        355                 360                 365

Phe Phe Arg Met Val Ile Ser Asn Pro Ala Ala Thr His Gln Asp Ile
    370                 375                 380

Asp Phe Leu Ile Glu Glu Ile Glu Arg Leu Gly Gln Asp Leu His His
385                 390                 395                 400

His His His His

## Description

### Technical Field

The present invention relates to a thermally stable glutamic acid decarboxylase 65 mutant and means for detecting and quantifying a human anti-GAD autoantibody that is useful in diagnosis of insulin-dependent diabetes mellitus (IDDM: Type I diabetes mellitus).

### Background Art

Glutamic acid decarboxylase (GAD) is an enzyme that decarboxylates glutamic acid to give GABA (γ-aminobutyric acid). GAD exists in GABAergic neuron nerve terminals in the brain, pancreatic islet β cells, or the like at high concentrations (see Bu, D. F., Erlander, M. G., Hitz, B. C., Tillakaratne, N. J. K., Kaufman, D. L., Wagner-McPherson, C. B., Evans, G. A. & Tobin, A. J. (1992) and Two human glutamate decarboxylase, 65-kDa Gad and 67-kDa GAD, are each encoded by a single gene. Proc. Natl. Acad. Sci. U.S.A., 89, 2115-2119). At the nerve ends, GAD exists within nerve terminal synaptic vesicles. In β cells, GAD exists bound to the cell membranes of synaptic-like microvesicles or in cytoplasm. GAD is intimately involved in GABA localization. It is thought that GABA in β cells participates in insulin synthesis or regulation of the functions of α and σ cells adjacent to β cells (see Christgau, S., Schierbeck, H., Aanstoot, H.-J., Aagaard, L., Begley, K., Kofod, H., Hejnaes, K. & Baekkeskov, S. (1991); Pancreatic β-cells express two autoantigenic forms of glutamic acid decarboxylase, a 65 kDa hydrophilic form and a 64 kDa amphiphilic form which can be both membrane-bound and soluble. J. Biol. Chem. 266, 21257-21264, Chrisgau, S., Aanstoot, H.-J., Schierbeck, H., Begley, K., Tullin, S., Hajnaes, H., & Baekkeskov, S. (1992); Membrane anchoring of the autoantigen GAD65 to microvesicles in pancreatic β-cells by palmitoylation in the N-terminal domain. J. Cell Biol. 118, 309-320, Reetz, A., Solimena, M., Matteoli, M., Folli, F., Takei, K. & DeCamilli, P. (1991); and GABA and pancreatic β-cells: colocalization of glutamic acid decarboxylase (GAD) and GABA with synaptic like microvesicles suggests their role in GABA storage and secretion, EMBOJ. 10, 1275-1284).

Human GAD includes two isoform types: GAD65, with a molecular weight of 65kDa, and GAD67, with a molecular weight of 67 kDa. GAD65 and GAD67 comprise 585 amino acids and 594 amino acids, respectively, and share 65% homology. GAD65 and GAD67 are governed by different genes located on chromosome 10 and chromosome 2, respectively. Both GAD65 and GAD67 are expressed in human brain and pancreatic islet β cells. GAD65 is predominantly expressed in β cells (see Bu, D. F., Erlander, M. G., Hitz, B. C., Tillakaratne, N. J. K., Kaufman, D. L., Wagner-McPherson, C. B., Evans, G. A. & Tobin, A. J. (1992); Two human glutamate decarboxylase, 65-kDa Gad and 67-kDa GAD, are each encoded by a single gene. Proc. Natl. Acad. Sci. U.S.A., 89, 2115-2119, Bu, D. F. & Tobin, A.J. (1994); The exon-intron organization of the genes (GAD1 and GAD2) encoding two human glutamate decarboxylases (GAD67 and GAD65) suggests that they derive from a common ancestral GAD, Genomics, 21, 222-228, Lernmark Å (1996); and Glutamic acid decarboxylase--gene to antigen to disease, J Intern. Med., 240, 259-277).

In recent years, GAD has been conventionally detected in patients with diabetes and discovered to be identical to an autoantigen generally referred to as the "64 autoantigen." Specifically, GAD has been identified as one of autoantigens deeply involved in insulin-dependent diabetes mellitus.

Insulin-dependent diabetes mellitus (IDDM; Type I diabetes mellitus) is developed by absolute deficiency of insulin due to selective disruption of pancreatic islet β cells by the autoimmune mechanism. An anti-GAD antibody is detected in patients with diabetes, and particularly in IDDM patients. However, the anti-GAD antibody also exists in the serum of the patients with non-insulin-dependent diabetes mellitus (NIDDM). Some NIDDM patients develop IDDM later. Furthermore, in the case of IDDM with delayed progression, such anti-GAD antibody is detected prior to the expression of the clinical signs of diabetes mellitus. In recent years, intermediate-type (between IDDM and NIDDM) diabetes mellitus referred to as Latent Autoimmune Diabetes in Adults (LADA) has also been reported. An anti-GAD antibody may also be detected in the case of this type of diabetes mellitus (Ikui et al., Diabetes Frontier,6, p. 404, 1995). An anti-GAD65 antibody can be confirmed at high frequencies in the serum of IDDM patients at time points several years before onset. Therefore, diabetes mellitus and particularly IDDM can be diagnosed or predicted via detection of such anti-GAD antibody. Diagnostic methods for diabetes mellitus, which are based on detection of such anti-GAD antibodies, are known (e.g., see WO92/04632 and WO92/03733). Specific methods employed in these documents are radioimmunoassay (RIA), enzyme-linked immunosorbent assay (ELISA), and the like. Selection of antigens and selection of detection means are very important in these assays in terms of sensitivity and specificity. It is known that anti-GAD antibodies in the serum of patients with diabetes mellitus and particularly patients with insulin-dependent diabetes mellitus mainly react with GAD65 (Ujihara et al., Diabetes, 43, p. 968, 1994).

Furthermore, the amino acid sequence of the GAD65 protein and the nucleotide sequence of the GAD 65 gene have already been known (see WO92/04632, Ding-Fang Bu, M. G. Erlander, B. C. Hitz, N. J. K. Tillakaratne, D. L. Kaufman, C. B. wagner-Mcpherson, G. A. Evans, A. J. Tobin (1992) and Two human glutamic decarboxylase, 65-kDa GAD and 67-kDa GAD, are each encoded by a single gene. Proc. Natl. Acad. U.S.A., 89, 2115-2119).

GAD65 was formerly extracted and purified from pig brain. However, such preparation involves contamination with GAD67. Hence, the use of such preparation as an antigen leads to lower assay specificity, meaning that correct measurement values cannot be obtained. Therefore, it has been expected in recent years that assay usefulness could be enhanced through the use of recombinant GAD65 prepared by a genetic engineering technique instead of the use of the above-mentioned natural extract (e.g., see JP Patent Publication (Kohyo) No. 5-503220 A (1993) (WO92/05446) and JP Patent Publication (Kohyo) No. 9-503387 A (1997) (WO95/07992)).

However, both GAD65 as a natural product and recombinant GAD65 have high hydrophobicity, so that the maintenance of the conformations thereof is difficult, they can easily become insoluble, the purification yields thereof are poor, and the maintenance of the antigenicity thereof is extremely difficult. Furthermore, it is known that the reactivity of the serum of a patient with Type I diabetes mellitus to GAD65 declines rapidly at room temperature. This may be because the conformation of GAD65 is thermally unstable as described above, or because an anti-GAD65 antibody in the serum of a patient precisely recognizes only the unaltered conformation of GAD65 (see Bu, D. F. & Tobin, A. J. (1994); The exon-intron organization of the genes (GAD1 and GAD2) encoding two human glutamate decarboxylases (GAD67 and GAD65) suggests that they derive from a common ancestral GAD. Genomics, 21, 222-228, Schwartz, H. L., Chandonia, J.-M., Kash, S. F., Kanaani, J. Tunnell, E., Domingo, A., Cohen, F. E., Banga, J. P., Madec, A.-M., Richter, W. & Baekkeskov, S. (1999); High-resolution autoreactive epitope mapping and structural modeling of the 65 kDa form of human glutamic acid decarboxylase. J. Mol. Biol., 287, 983-999, Ding-Fang Bu, M. G. Erlander, B. C. Hitz, N. J. K. Tillakaratne, D. L. Kaufman, C. B. wagner-Mcpherson, G. A. Evans, A. J. Tobin (1992); and Two human glutamic decarboxylase, 65-kDa GAD and 67-kDa GAD, are each encoded by a single gene. Proc. Natl. Acad. U.S.A., 89, 2115-2119).

The low thermal stability of the conformation of GAD65 makes the production of GAD65 with antigenicity difficult. Moreover, such low thermal stability leads to a lack of sensitivity and accuracy in detection or quantification of an autoantibody in the serum of a patient as described above, This results in undermined reliability of IDDM diagnosis using GAD65.

Meanwhile, yeast (Pichia and Saccaromyces) and *in vitro* expression are currently mainly used for recombinant expression systems for GAD65 (see Law, R. H. P., Rowley, M. J., Mackay, I. R. & Corner, B. (1998) and Expression in Saccharomyces cerevisiae of antigenially and enzymatically active recombinant glutamic acid decarboxylase. J. Biotechnol, 61, 57-68). However, these expression systems are all inferior to expression systems that use bacteria due to complexity of procedures, length of culture time, and economic inefficiency.

Furthermore, human GAD65 is an insoluble protein. Human GAD65 in an insoluble form is separated, purified, or the like with difficulty. Moreover, if human GAD65 is solubilized using a solubilizing agent such as a surfactant, human GAD65 will lose antigenicity due to protein denaturation and the like. However, no successful expression of GAD65 alone in a soluble form in bacteria such as *Escherichia coli* has been reported.

### Disclosure of the Invention

An object of the present invention is to obtain thermally stable GAD65 mutants retaining antigenicity to pancreatic islet-β-cell-specific autoantibodies in the serum of a patient. Alternatively, objects of the present invention are to solubilize the GAD65 mutants while retaining the antigenicity and thermal stability thereof and to provide a novel means for detecting and quantifying pancreatic islet-β-cell-specific autoantibodies in the serum of a patient with better accuracy and sensitivity using these GAD65 mutants.

To achieve the above objects, the present inventors have constructed, based on a molecular evolution engineering technique, an experimental evolution system relating to thermal stability using GAD65 antigenicity to the serum of a patient as an indicator. The present inventors have introduced random mutations into the above GAD65 genes, caused the expression of these mutant genes, obtained many GAD65 protein mutants, and then screened for these protein mutants in view of thermal stability and antigenicity. As a result, the present inventors have finally obtained thermally stable GAD65 protein mutants retaining their antigenicity to the serum of a patient. The present inventors have further succeeded in separation and purification of solubilized GAD65 protein mutants retaining thermal stability and antigenicity. Moreover, the present inventors have also confirmed that a detection and quantification system for β-cell-specific autoantibodies using these protein mutants has better sensitivity and accuracy. Thus, the present inventors have completed the present invention.

The present invention is as described below.
(1) A glutamic acid decarboxylase 65 mutant, which has a mutation in the region between amino acid residues 310 and 470 in the amino acid sequence represented by SEQ ID NO: 2 in Sequence Listing and antigenicity to a pancreatic islet-β-cell-specific autoantibody and retains 50% or more of the relative antigenicity when heated at 45°C for 10 minutes.
(2) A glutamic acid decarboxylase 65 mutant, which has any one mutation of the following amino acid mutations (a) to (f) in the amino acid sequence represented by SEQ ID NO: 2:
   (a) E412G;
   (b) S313F;
   (c) M361I and/or F467S;
   (d) K355R;
   (e) D345V; and
   (f) I353T.
(3) A glutamic acid decarboxylase 65 mutant, which lacks a region on the N terminal side of the glutamic acid decarboxylase 65 mutant according to (2) above and is solubilized.
(4) The glutamic acid decarboxylase 65 mutant according to (3) above, which lacks amino acid residues 1 to 187.
(5) A glutamic acid decarboxylase 65 mutant, in which thioredoxin is added to the N terminus of the glutamic acid decarboxylase 65 mutant according to any one of (1) to (3) above.
(6) A reagent for detection or quantification of a pancreatic islet-β-cell-specific autoantibody, which contains the glutamic acid decarboxylase 65 mutant according to any one of (1) to (5) above.
(7) A diagnostic agent for insulin-dependent diabetes mellitus, which contains the glutamic acid decarboxylase 65 mutant according to any one of (1) to (5) above.
(8) A DNA, which has a nucleotide sequence encoding the glutamic acid decarboxylase 65 mutant according to any one of (1) to (5) above.
(9) A DNA, which has any one of the following mutations (a) to (f) in the nucleotide sequence represented by SEQ ID NO: 1:
   (a) a mutation wherein GAG between nucleotides 1234 and 1236 in the nucleotide sequence represented by SEQ ID NO: 1 is substituted with GGA, GGC, GGG, or GGT;
   (b) a mutation wherein TCT between nucleotides 937 and 939 in the aforementioned nucleotide sequence is substituted with TTC or TTT;
   (c) a mutation wherein ATG between nucleotides 1081 and 1083 in the aforementioned nucleotide sequence is substituted with ATA, ATC, or ATT, and/or TTT between nucleotides 1399 and 1401 in the aforementioned nucleotide sequence is substituted with AGC, AGT, TCA, TCC, TCG, or TCT;
   (d) a mutation wherein AAA between nucleotides 1063 and 1065 in the aforementioned nucleotide sequence is substituted with AGA, AGG, CGA, CGC, CGG, or CGT;
   (e) a mutation wherein GAC between nucleotides 1033 and 1035 in the aforementioned nucleotide sequence is substituted with GTA, GTC, GTG, or GTT; and
   (f) a mutation wherein ATT between nucleotides 1057 and 1059 in the aforementioned nucleotide sequence is substituted with ACA, ACC, ACG, or ACT.
(10) The DNA according to (9) above, which lacks a nucleotide sequence ranging from nucleotides 1 to 561 in the nucleotide sequence represented by SEQ ID NO: 1.
(11) The DNA according to (9) or (10) above, which has the nucleotide sequence represented by SEQ ID NO: 27 on the 5' terminal side.
(12) An antisense DNA or an antisense RNA, which forms an antisense strand in relation to the DNA according to any one of (8) to (11) above.
(13) A recombinant vector, into which the DNA according to any one of (8) to (11) above is inserted.
(14) A transformant, which is transformed with the recombinant vector according to (13) above.
(15) A method for producing a glutamic acid decarboxylase 65 mutant, which comprises culturing the transformant according to (14) above in medium and then harvesting the glutamic acid decarboxylase 65 mutant according to any one of (1) to (5) above from the culture product.

This description includes part or all of the contents as disclosed in the description and/or drawings of Japanese Patent Application No. 2005-074056, which is a priority document of the present application.

### Brief Description of the Drawings

Fig. 1 shows an outline of a screening system for the GAD65 mutants and the genes thereof of the present invention.
Fig. 2 shows the results of selecting and observing clones using the screening system of the present invention and direct immunostaining.
Fig. 3 shows schematic diagrams showing the relationship between mutation positions of the GAD65 mutants of the present invention and the conformation of GAD65. Fig. 3A shows the central region (dimer) of GAD65. Fig. 3B shows the C-terminal region of GAD65.
Fig. 4 shows an outline of a PCR method that uses mutation primers.
Fig. 5 shows an outline of procedures for preparing a plate for immobilization of a thermally stable GAD65 mutant antigen.
Fig. 6 shows an outline of the procedures of a method for measuring an anti-GAD antibody.
Fig. 7 is a graph showing the dilution curves of patients' serum specimens positive for an anti-GAD antibody obtained when a GAD65-Δ95 antigen and a GAD65-Δ187 mutant strain F86 antigen were separately used.
Fig. 8 is a graph showing the results of measuring the antigenicity of the GAD65-Δ187 mutant of the present invention.
Fig. 9 is a graph showing the results of measuring the thermostability of the GAD65-Δ187 mutant of the present invention.
Fig. 10 is a graph showing the results of measuring fluctuations in antigenicity resulting from lengthy heat treatment performed on the GAD55-Δ187 mutant of the present invention.
Fig. 11 is a graph showing the results of measuring fluctuations in antigenicity resulting from high-temperature (60°C) heat treatment performed on the GAD65-Δ187 mutant of the present invention.
Fig. 12 is a graph showing the results of measuring fluctuations in antigenicity resulting from heat treatment performed on the GAD65-Δ187 mutant of the present invention in the presence of 10% methanol.
Fig. 13 shows an outline of the structure of each expression plasmid constructed in a solubility test in Example 8 and techniques for constructing the same.
Fig. 14 is a graph showing an anti-GAD antibody standard calibration curve that was obtained from the results of measuring changes in luminescence intensity based on binding between serum positive for GAD (with various concentrations) of a patient and the GAD65 mutant of the present invention.
Fig. 15 shows the multiple cloning sites of a pThioHisA vector.

### Best Mode for Carrying Out the Invention

The GAD65 mutants of the present invention have antigenicity to pancreatic islet-β-cell-specific autoantibodies and retain 50% or more of their relative antigenicity when heated at 45°C for 10 minutes. The relative antigenicity is represented by the proportion (percentage) of the reactivity of a mutant enzyme to an anti-GAD65 antibody when heated with respect to the reactivity of the mutant enzyme to the same when not heated.

The thermostable GAD65 mutants and the genes thereof of the present invention are obtained by introducing random (1 to several) mutations into each GAD65 gene through application of an error-prone PCR method using the GAD65 gene (SEQ ID NO: 1) as a template and a small amount of manganese chloride (MgCl₂), constructing a random library containing many mutant genes, causing the expression of the random library by *Escherichia coli,* and then screening for clones expressing proteins that exert antigenicity and thermostability.

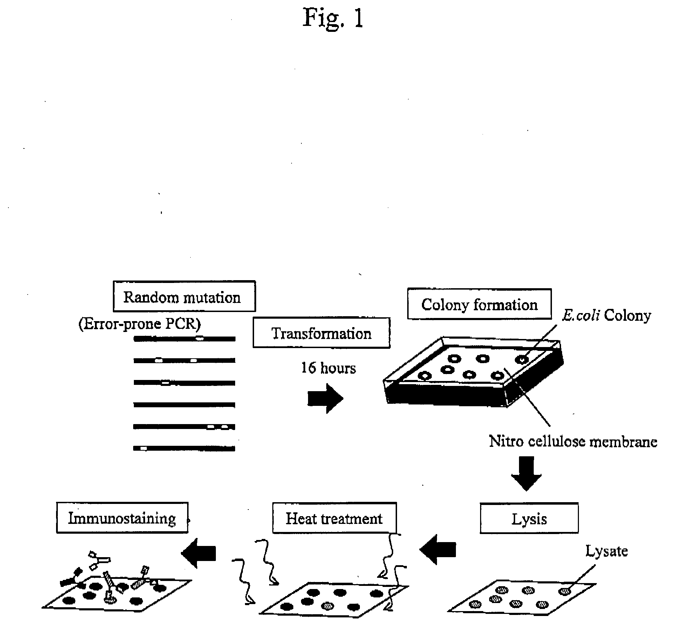

Such screening system for the GAD65 mutants and the genes thereof of the present invention will be further specifically explained with reference to Fig. 1.

As the concentration of manganese chloride that is used in the above error-prone PCR method increases, the resulting mutation frequency increases. With 50 µM manganese chloride, the number of nucleotides substituted per cDNA ranged from 1 to 3 and was 1.5 in average. With 70 µM manganese chloride, the number of nucleotides substituted per cDNA ranged from 0 to 4 and was 2.3 in average. To clarify the relationship between the thermostability of a GAD65 mutant and the mutation causing such thermostability, the number of nucleotides substituted is preferably approximately 1 to 2 on the average. As described above, the concentration of manganese chloride is regulated.

The GAD65 gene to be used as a template may be a full-length gene or a gene lacking the nucleotide sequence encoding a region on the N-terminal side of the GAD65 protein as described later for enhancement of solubilization. Moreover, such gene may be in a form incorporated in a plasmid or the like, as long as it is in the form of DNA containing such gene region. Upon screening in the present invention, a mutant gene random library obtained by the error-prone PCR method using such GAD65 gene as a template is incorporated into a vector. A host microorganism such as *Escherichia coli* is transformed using the recombinant vector. For example, the thus obtained transformant is cultured after placing a nitro cellulose membrane on an LB plate coated with an IPTG solution and then inoculating the same thereon. Thus, colonies are formed. Subsequently, replicas of colonies are formed on a nitro cellulose membrane. Microbial bodies on the replicas are lysed, immobilized, and then subjected to heat treatment at 45°C for 15 minutes, for example. Next, the nitro cellulose membrane is subjected to direct immunostaining.

When such immunostaining is performed, a nitro cellulose membrane (on which the microbes have been lysed) is immersed in an anti-GAD65 primary antibody solution, followed by 1 hour of reaction at 25°C, for example. Furthermore, the solution is caused to react with a solution containing a labeled secondary antibody (corresponding to the primary antibody). The reaction may be performed using a labeled anti-GAD65 primary antibody alone. Examples of labels to be bound to antibodies include horseradish peroxidase (HRP) and alkaline phosphatase (ALP).

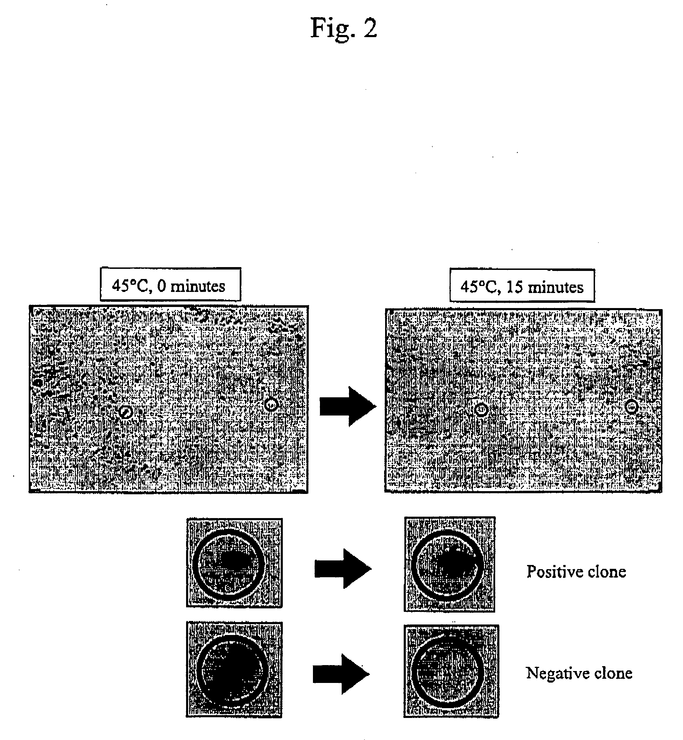

Colonies intensely stained on a nitro cellulose membrane after these procedures are formed by clones exerting antigenicity (see Fig. 2).

Colonies on a nitro cellulose membrane is derived from the replica of an original nitro cellulose membrane. Original colonies corresponding to the above clones exerting antigenicity are formed by clones expressing a target GAD65 mutant exerting thermostability and antigenicity. These clones are cultured and then the GAD65 mutant can be separated and purified from the culture product containing microbial bodies. Moreover, such mutant gene can be easily obtained by harvesting introduced recombinant vectors from microbial bodies and then treating them with restriction enzymes, for example. Moreover, such mutant gene can also be easily obtained through PCR amplification of the GAD65 mutant gene region using the above recombinant vector as a template.

The GAD65 mutants of the present invention are each insoluble because hydrophobic amino acids are abundantly located in a region on the N-terminal side. Therefore, it is desired that the GAD65 mutants be solubilized to facilitate purification thereof and the like. For this purpose, a fusion protein is prepared by adding thioredoxin (SEQ ID NO: 28) to a region on the N-terminal side of each GAD65 mutant. Alternatively, a region on the N-terminal side may be deleted from the GAD65 mutant. Furthermore, a combination of these means may be employed.

Furthermore, a polyhistidine tag may also be added to the C-terminal side of GAD65 mutants of the present invention. A protein to which a polyhistidine tag has been added can be purified extremely easily through the use of a purification means (e.g., a Ni⁺⁺ sepharose column) carrying metal ions (e.g., Ni⁺⁺ or Co⁺⁺) that form complexes with polyhistidine.

To obtain such fusion protein, a host microorganism such as *Escherichia coli* is transformed with a vector. This vector has been constructed so that a thioredoxin gene (SEQ ID NO: 27) is located on the 5' upstream side of a GAD65 mutant gene or the gene lacking the nucleotide sequence corresponding to the amino acid sequence of a region on the N-terminal side of the GAD65 mutant. Alternatively, this vector may have been constructed so that the nucleotide sequence corresponding to a polyhistidine tag is located on the 3' downstream side of the GAD65 mutant gene in addition to the inclusion of the thioredoxin gene on the 5' upstream side. The thus transformed *Escherichia coli* is then cultured. Regarding the thus obtained GAD65 mutant, increases in soluble protein expression levels can be clearly confirmed without the generation of a by-product, a peptide (48 kDa), that is generated due to mis-transcription of amino acid 165 and the following amino acids of GAD65 as seen in the cases of conventional technology (M. L. Papouchado, S. N. Valdez, D. Ghiringhelli, E. Poskus, M. R. Ermacora, (1997). Expression of properly folded human glutamate decarboxylase 65 as a fusion protein in Escherichia coli. Eur. J. Biochem. 246, 350 to 359).

The terms "thioredoxin" and "the gene thereof" in the present invention encompasses a partial peptide on the N-terminal side of thioredoxin and DNA encoding the same.

A specific example of a vector to be used herein is pThioHisA.

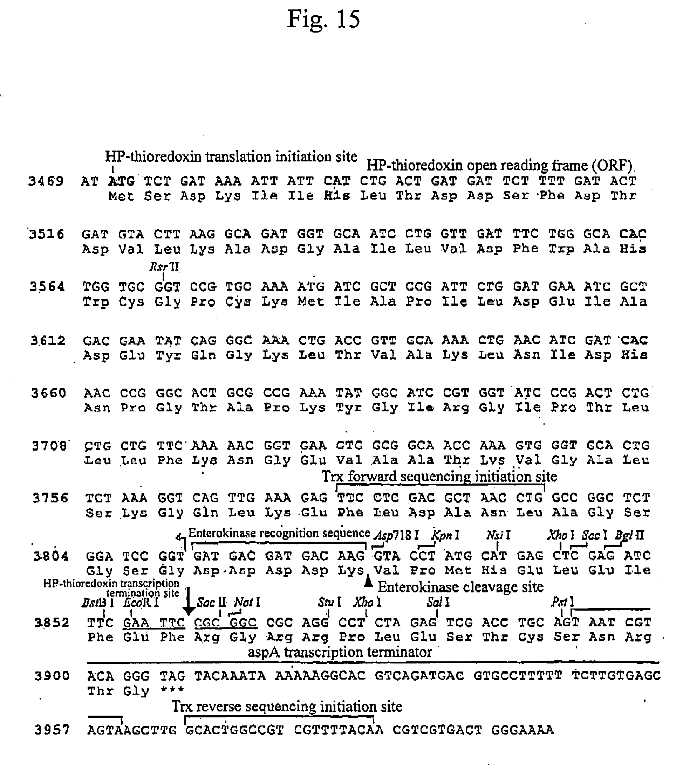

Fig. 15 shows the multicloning sites (SEQ ID NOS: 29 and 30) of the vector. The GAD65 mutant gene of the present invention or a gene lacking the nucleotide sequence corresponding to the amino acid sequence of a region on the N-terminal side of the GAD65 mutant is inserted into the *Eco*R I*-Not* I site at the position indicated by an arrow in Fig. 15.

In the present invention, amino acids 1 to 187 on the N-terminal side of the full-length GAD65 protein (SEQ ID NO: 2) were deleted and then thioredoxin and a histidine tag were added to the N terminus and the C terminus, respectively, thereby preparing fusion proteins as the GAD65 mutants. As a result of screening with the above techniques, the following GAD65 mutants having good thermostability and high antigenicity were obtained.
a (F52); E412G (SEQ ID NO: 6)
b (F68); S313F (SEQ ID NO: 8)
c (F1); M361I, F467S (SEQ ID NO: 10)
d (F86); K355R (SEQ ID NO: 12)
e (F87); D345V (SEQ ID NO: 14)
f (F84); I353T (SEQ ID NO: 16)

In addition, alphabetical characters and numerals shown in parentheses following "a" to "f" above are used to indicate the names of mutants. Specifically, first alphabetical characters (capital letters) and numerals following semicolon above indicate amino acid residues before mutation in wild-type GAD65 represented by SEQ ID NO: 1 and their positions, respectively. Alphabetical characters (capital letters) at the ends indicate amino acid residues after mutation at the same positions.

Test results for the above mutants described later in Examples demonstrate that the mutants possess good thermostability and antigenicity effective for the serum of a patient with type I diabetes mellitus, although they lack at least amino acid residues 1 to 187 on the N-terminal side of the amino acid sequence.

Such mutants lacking amino acid residues 1 to 187 on the N-terminal side of the amino acid sequence have antigenicity to a GAD65 antibody in the serum of such patient. Accordingly, a GAD65 mutant lacking a portion comprising less than 187 amino acid residues or a mutant with no such deficiency contains the entire epitope portion of the mutant lacking amino acid residues 1 to 187 on the N-terminal side and has antigenicity.

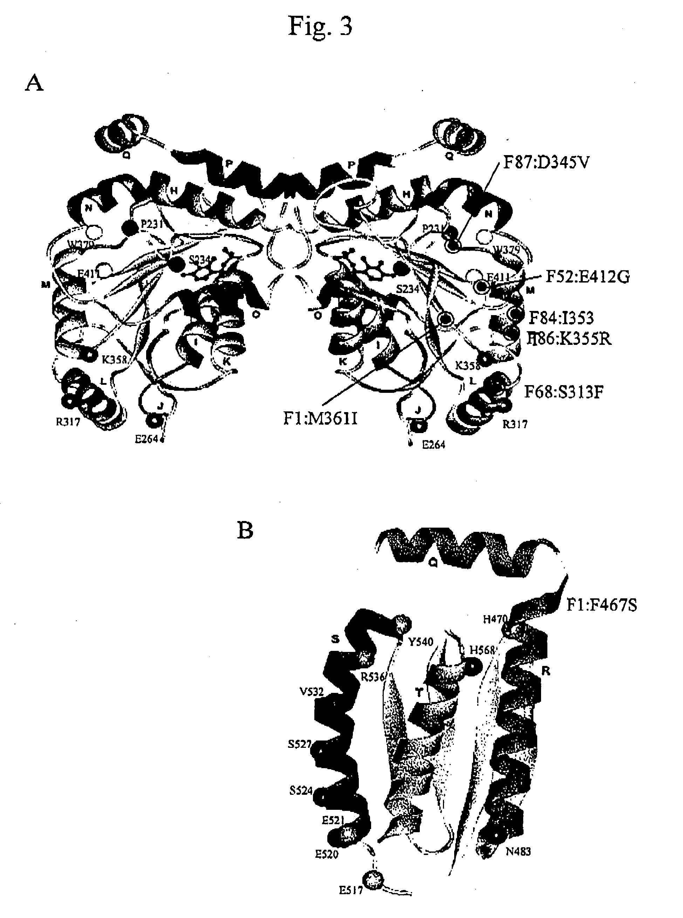

Fig. 3 shows the relationship between presumed GAD65 conformations and amino acid mutation positions in the above mutants. As shown in Fig. 3, amino acid mutation positions in the above mutants are located in the middle domains (indicated with M in Fig. 3) of α-helix structures and regions (thought to contribute to the stability of the α-helix structures) following or enclosing such middle domains. It is thus understood that amino acid mutations in such regions are particularly important for thermostability. The regions are expressed with position numbers in the amino acid sequence of GAD65. Such a region is between amino acid residues 310 and 470, particularly between amino acid residues 313 and 467, and further specifically between amino acid residues 319 and 412. In addition, F1 also has a mutation in the R region, in addition to a mutation in the vicinity of the M region as described above. Furthermore, the mutation (F467S) in the R region of F1 suggests the presence of other regions contributing in an additive manner to heat resistance based on mutation positions of other mutants. However, this may not be a major cause.

Therefore, according to the present invention, the GAD65 mutant of the present invention is a mutant having a mutation in the region between amino acid residues 310 and 470 of the amino acid sequence represented by SEQ ID NO: 2 and (i) antigenicity to a pancreatic islet-β-cell-specific autoantibody and (ii) retaining 50% or more of relative antigenicity when the mutant is heated at 45°C for 10 minutes.

Examples of the above mutations include any one of specific mutations "a" to "f" above and/or "other mutations" that satisfy the characteristics (i) and (ii) above. Here, examples of such "other mutations" include, in the region between amino acid residues 310 and 470, substitution, deletion, or addition of one or several amino acid residues and preferably substitution of the same, for example. "Several" means integers between 2 and approximately 10. In particular, "substitution" means substitution of amino acids that are analogous or to differ from each other in terms of physicochemical properties such as structure and polarity. When the above mutations are composed of only "other mutations" as described above, the preferable substitution is a substitution of amino acids that differ in physicochemical properties. In contrast, when the above mutations are composed of any one of the specific mutations "a" to "f" above and "other mutations" as described above, the preferable substitution is a substitution of amino acids that are analogous in terms of physicochemical properties. Examples of amino acids that are analogous to each other in terms of physicochemical properties include: nonpolar amino acids such as Gly, Ala, Val, Leu, Ile, and Pro; polar non-charged-type amino acids such as Ser, Thr, Met, Asn, GIn, and Cys; aromatic amino acids such as Phe, Tyr, and Trp; negatively charged amino acids such as Asp and Glu; or positively charged amino acids such as Lys, Arg, and His. Examples of amino acids that differ from each other in terms of physicochemical properties include amino acids each having different properties such as polar non-charged type amino acids and aromatic amino acids, nonpolar amino acids and charged type amino acids, and nonpolar amino acids and aromatic amino acids. Amino acids to be substituted may be not only L-amino acids, but also D-amino acids. Examples of "other mutations" include, but are not limited to, F344S, 1358V, S436P, and M415L.

Alternatively, other examples of the above mutations may include specific mutations in the region between amino acid residues 310 to 470 described above (e.g., any one of the specific mutations "a" to "f' above) and "other mutations" that satisfy the characteristics of (i) and (ii) above. Here, one of "other mutations" means substitution, deletion, or addition of one or several amino acid residues, such as in a region other than the region between amino acid residues 310 and 470, and preferably substitution of the same. "Substitution" used herein means substitution of amino acids that are analogous or differ from each other in terms of physicochemical properties. Examples of such amino acid types are as illustrated above. Examples of "other mutations" include, but are not limited to, V219A, Y480C, C304R, P260L, D552V, E489Y, H568L, L516P, V219A, F259I, E520G, and M493V.

The nucleotide sequences of the mutant genes corresponding to the above GAD65 mutants "a" to "f" obtained according to the present invention have mutations shown below with respect to the nucleotide sequence of the wild-type GAD65 gene represented by SEQ ID NO: 1.
(a) GAG→GGG (A→G) (nucleotides 1234 to 1236 in the nucleotide sequence)
(b) TCT→TTT (C→T) (nucleotides 937 to 939 in the nucleotide sequence)
(c) ATG→ATA (G→A) (nucleotides 1081 to 1083 in the nucleotide sequence)
   TTT→TCT (T→C) (nucleotides 1399 to 1401 in the nucleotide sequence)
(d) AAA→AGA (A→G) (nucleotides 1063 to 1065 in the nucleotide sequence)
(e) GAC→GTC (A→T) (nucleotides 1033 to 1035 in the nucleotide sequence)
(f) ATT→ACT (T→C) (nucleotides 1057 to 1059 in the nucleotide sequence)

Table 1 shows a summary of the relationships between these nucleotide sequence mutations and amino acid mutations.

**Table 1**

| | Clone # | Amino acid | | | Nucleotide | | | | | | |
|---|---|---|---|---|---|---|---|---|---|---|---|
| | | Mutation position | Before mutation | After mutation | Position of codon for mutation | DNA | | Mutation of nucleic acid | RNA | | Mutation of nucleic acid |
| | | | | | | Codon (before mutation) | Codon (after mutation) | | Codon (before mutation) | Codon (after mutation) | |
| a | F52 | 412 | Glu | Gly | 1234 - 1236 | GAG | GGG | A→G | GAG | GGG | A→G |
| b | F68 | 313 | Ser | Phe | 937 - 939 | TCT | TTT | C→T | UCU | UUU | C→U |
| c | F1 | 361 | Met | Ile | 1081 - 1083 | ATC | ATA | G→A | AUG | AUA | G→A |
| | | 467 | Phe | Ser | 1399 - 140I | TTT | TCT | T→C | UUU | UCU | U→C |
| d | F86 | 355 | Lys | Arg | 1063 - 1065 | AAA | AGA | A→G | AAA | AGA | A→G |
| e | F87 | 345 | Asp | Val | 1033 - 1035 | GAC | GTC | A→T | GAC | GUC | A→U |
| f | F84 | 353 | Ile | Thr | 1057 - 1059 | ATT | ACT | T→C | AUU | ACU | U→C |

Not all the codons encoding the above mutated amino acid residues are listed in Table 1. In the present invention, mutant genes having codons that encode mutated amino acid residues listed in Table 1 are also encompassed. Specifically, DNAs that undergo the following mutations in the nucleotide sequence represented by SEQ ID NO: 1 are encompassed herein.
(a) Nucleotides 1234 to 1236 in the nucleotide sequence, which cause E412G, are GGA, GGC, GGG, or GGT.
(b) Nucleotides 937 to 939 in the nucleotide sequence, which cause S313F, are TTC or TTT.
(c) Nucleotides 1081 to 1083 in the nucleotide sequence, which cause M361I, are ATA, ATC, or ATT and
   nucleotides 1399 to 1401 in the nucleotide sequence, which cause F467S, are AGC, AGT, TCA, TCC, TCG, or TCT.
(d) Nucleotides 1063 to 1065 in the nucleotide sequence, which cause K355R, are AGA, AGG, CGA, CGC, CGG, or CGT.
(e) Nucleotides 1033 to 1035 in the nucleotide sequence, which cause D345V, are GTA, GTC, GTG, or GTT.
(f) Nucleotides 1057 to 1059 in the nucleotide sequence, which cause 1353T, are ACA, ACC, ACG, or ACT.

Furthermore, antisense DNAs and antisense RNAs that compose antisense strands in relation to these DNAs are also encompassed herein.

In the present invention, a gene expressing full-length GAD65 having an amino acid mutation as described above and a gene expressing a GAD65 mutant lacking a region on the N-terminal side can also be easily obtained with the use of a PCR method using mutation primers, a cassette conversion method using synthetic DNA, or the like.

Examples of such methods include the following methods. However, other methods can also be employed herein and the examples are not limited to these examples.

### (1) PCR method using mutation primers

A gene that expresses full-length GAD65 having no mutations or a gene that expresses GAD65 lacking a region on the N-tenninal side is inserted into a recombinant vector. Meanwhile, a PCR forward primer (primer F1) having the nucleotide sequence of a cleavage site of a restriction enzyme (restriction enzyme "x," e.g., *Eco*R I) located at a multicloning site of the vector and a PCR reverse primer (primer R1) having the nucleotide sequence of a cleavage site of another restriction enzyme (restriction enzyme "y," e.g., *Not* I) located on the 3' side (on the multicloning site) from the restriction enzyme of the forward primer are prepared. Furthermore, a PCR forward primer of at least 20 nucleotides in length (primer F2) having target mutation nucleotides in the center of a site into which a mutation is inserted and a PCR reverse primer (primer R2) complementary to the primer F2 are prepared. These primers are synthesized by a DNA synthesis method.

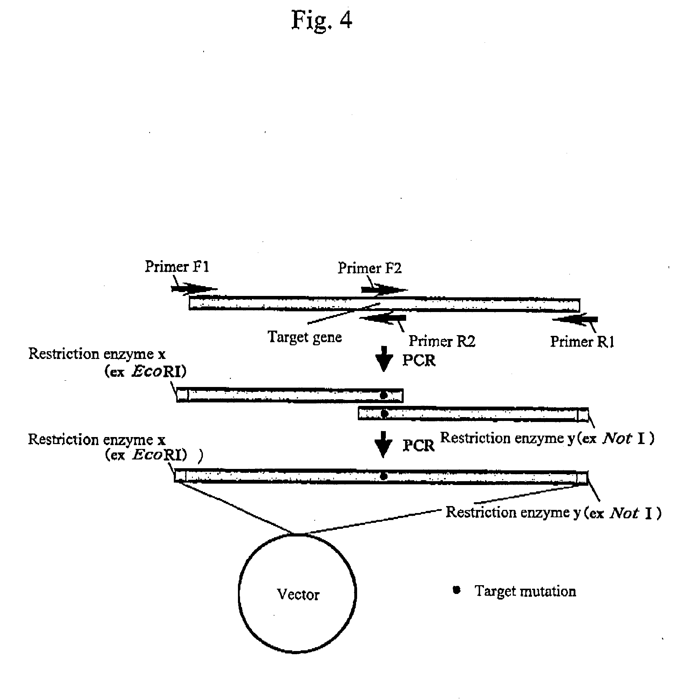

With the use of DNA that expresses full-length GAD65 having no mutations in the nucleotide sequence or DNA that expresses GAD65 lacking a region on the N-terminal side as a template and primers F1 and R2 or primers F2 and R1, a 5' fragment and a 3' fragment of the target mutant gene are amplified by the PCR method. The two types of fragment are separately isolated and then mixed. The mutant gene is amplified by the PCR method. Fig. 4 shows an outline of the procedures. In addition, subsequently, a greater amount of the mutant gene can also be acquired by performing PCR using the mutant gene as a template and primers F1 and R1.

Next, the full-length GAD65 mutant into which the target mutation has been introduced or the GAD65 mutant gene lacking the region on the N-terminal side is digested with restriction enzymes "x" and "y." In the meantime, the original vector is also digested with the restriction enzymes "x" and "y." The thus obtained vector DNA fragment and the mutant gene fragment are linked using DNA ligase, so that a vector having the mutant gene as a result of introduction of the target mutation can be constructed.

### (2) Cassette conversion method using synthetic DNA

A gene that expresses full-length GAD65 having no mutations or a gene that expresses GAD65 lacking a region on the N-terminal side is inserted into a recombinant vector. Digestion with restriction enzymes is performed at restriction enzyme sites located upstream and downstream of a site to which nucleotides are inserted for mutation. A gene (cassette-removed gene) from which a normal-sequence nucleotide fragment has been removed is prepared. Meanwhile, a nucleotide fragment (cassette to be inserted) having a target mutation and the above restriction enzyme sites on both ends is synthesized by a DNA synthesis method. The mutation nucleotide fragment (cassette to be inserted) and the above gene (cassette-removed gene) are linked using DNA ligase. Thus, a vector having the mutant gene that expresses full-length GAD65 in which the target mutation has been inserted and the mutant gene that expresses GAD65 lacking the region on the N-terminal side can be constructed. In addition, such series of procedures can also be performed after separate preparation of a target gene in advance through digestion with restriction enzymes "x" and "y" in (1) above. In such case, the target gene can be finally inserted into a vector using restriction enzyme sites "x" and "y."

The GAD65 mutants of the present invention are effective for detection or quantification of autoantibodies that are generated along with disruption of particularly pancreatic islet β cells. Hence, the GAD65 mutants are useful for diagnosis of insulin-dependent diabetes mellitus (IDDM) and detection of preclinical conditions thereof. Diagnostic methods for IDDM based on detection of anti-GAD antibodies are broadly known. Examples of specific measurement methods include radioimmunoassay (RIA) and enzyme-linked immunosorbent assay (ELISA).

A preferable method for detection or quantification of the above autoantibodies of patients using the GAD65 mutants of the present invention is an ELISA method. A method particularly preferably used herein is a chemiluminescence ELISA method having sensitivity and specificity better than the RIA method in terms of performance. The chemiluminescence ELISA method is as described below.

The chemiluminescence ELISA method is an ELISA method for detecting or measuring an anti-GAD antibody, which comprises causing a sample to come into contact with a carrier on which a thermally stable GAD65 mutant antigen has been immobilized, binding the antigen with an antibody within the sample to form a complex, and detecting the complex using chemiluminescent dioxycetane having a group that can be cleaved with an enzyme and an antibody labeled with the enzyme that cleaves the group to generate chemiluminescence.

### 1) ELISA

ELISA (enzyme-linked immunosorbent assay) is a general analysis method that is broadly employed for detection, quantification, and the like of substances in a living body 1 and the like. In the present invention, an antigen (GAD65 mutant antigen) corresponding to an antibody (human anti-GAD antibody) to be detected is bound to an appropriate carrier in advance and then a sample is reacted with the carrier. If an anti-GAD antibody is present in the sample, it binds to the carrier via the GAD65 mutant antigen. Subsequently, an enzyme-labeled anti-human immunoglobulin antibody binds to the anti-GAD antibody that has been bound to the carrier and then the label is detected. A human anti-GAD antibody can be measured in this manner.

In the present invention, it is preferable to use a plate on which a GAD65 mutant antigen has been immobilized. A sample to be analyzed (appropriately diluted or undiluted serum, plasma, blood, or the like) containing an anti-GAD antibody is added to such plate, so that the anti-GAD antibody binds to the GAD65 mutant antigen. Subsequently, the plate is washed, thereby separating and removing components that have remained unbound to the GAD antigen. Next, an appropriate amount of an enzyme-labeled anti-human immunoglobulin antibody is added so that the antibody binds to the anti-GAD antibody. The plate is then washed, thereby separating and removing excessive enzyme-labeled anti-human immunoglobulin antibody that has remained unbound to the anti-GAD antibody. Furthermore, an appropriate enzyme substrate is added and then the finally generated enzyme reaction product is measured. The concentration of the anti-GAD antibody in a specimen to be analyzed can be measured. When GAD-immobilized particulates or the like are used as carriers, the concentration of an anti-GAD antibody can be measured by a similar method.

A competitive ELISA method is also known as another method. In this case, an enzyme-labeled anti-GAD antibody is allowed to co-exist with an anti-GAD antibody in a sample, so that they are caused to compete over binding to a GAD65 mutant antigen. Thereafter, a decrease in the binding level of the labeled anti-GAD antibody due to binding of the anti-GAD antibody in the sample is measured.

### 2) Reagents to be used herein and the like

### i. Thermally stable GAD65 mutant

The thermally stable GAD65 mutant antigens of the present invention can be used in a form bound to carriers. Many types of carriers suitable for ELISA are known. Examples of such carriers include glass, polystyrene, polyvinyl chloride, polypropylene, polyethylene, polycarbonate, dextrin, nylon, amylose, natural cellulose, modified cellulose, polyacrylamide, agarose, and magnetite. Examples of the forms of such carriers include microtiter plates, particulates, and beads. Examples of commercial products of these carriers include a "FluoroNunc module plate (produced by Nagle Nunc International, K.K.)" and "Opaque Stripwell (produced by Costar)." Furthermore, examples of commercial products of particulates include "Polybead-Polystyrene Microspheres (magnetic polystyrene microsphere) (produced by Polyscience, Inc.)," and "Estapor (magnetic particulates and non-magnetic particulates) (produced by Prolabo)." Furthermore, examples of commercial products of beads include "hydrazide beads (produced by Pierce)."

A carrier to which a thermally stable GAD65 mutant antigen has been immobilized (bound) can be prepared by directly binding the antigen onto the carrier or binding the same via another substance.

An example of such method for direct binding is a method that involves adding an appropriate amount of a GAD solution with an appropriate concentration into each well of a plate and then causing the plate to stand overnight.

An example of such method for binding via another substance is a method that involves binding via a cross linking agent or an anti-GAD antibody. For example, an appropriate amount of an anti-GAD antibody solution with an appropriate concentration is added to each well of a plate, the plate is allowed to stand overnight, a GAD solution with an appropriate concentration is added to each well of the plate, and then the plate is allowed to stand overnight. Thus, the GAD antigen can be bound to the plate.

The amount of the relevant GAD65 mutant that is immobilized as an antigen on a carrier may be at any level unless the luminescence intensity of a chemiluminescent substance used in the detection hits the peak. Specifically, a preferable amount of a GAD antigen obtained from the mutant ranges from approximately 0.1 to 2.0 µg (the amount of a protein as a GAD antigen; measurement value obtained by the Bradford method)/well.

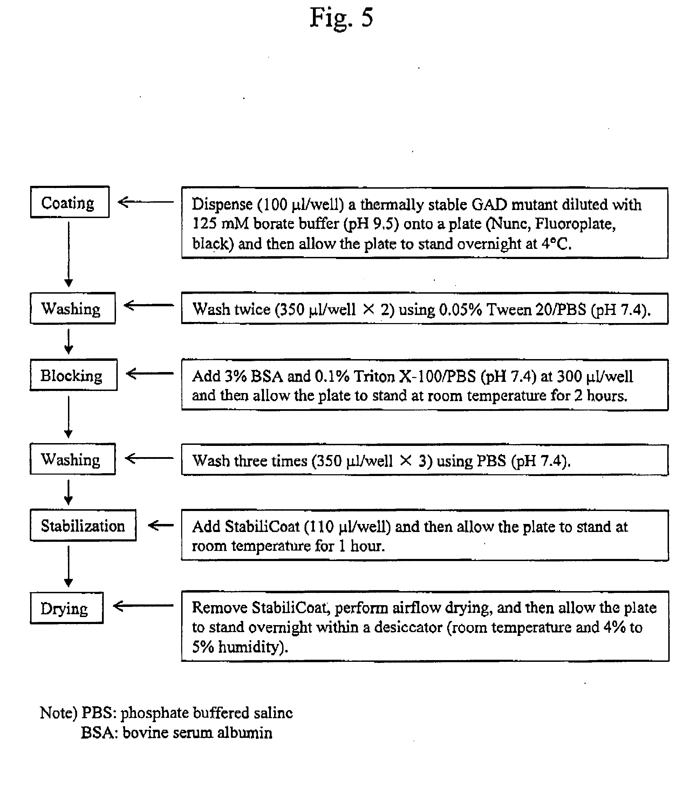

Fig. 5 shows an example of procedures for preparing a carrier (to which a GAD65 mutant antigen is immobilized by direct binding), which is employed in the present invention. Such carrier to which the GAD has been immobilized can be prepared when used or may be prepared in advance and then stored for an appropriate time. Storage conditions and the like for such carrier are known by persons skilled in the art. For example, a carrier to which such GAD antigen has been immobilized is dried or can be stably stored at 4°C to -20°C, for example, without drying.

### ii. Chemiluminescent dioxycetane

As chemiluminescent dioxycetane to be used in the present invention, substances disclosed in WO88/00695, JP Patent Publication (Kokai) No. 8-13406 A (1996), and patent application relating thereto can be used. Specifically, 2-chloro 5-(4-methoxysipiro {1,2-dioxycetane-3,2'-(5'-chloro)-tricyclo [3.3.1.1^{3,7}]decane}-4-yl)-1-disodium phenyl phosphate (commercial name: "CDP-Star (Trademark)") and 3-(4-methoxyspiro {1,2-dioxycetane-3,2'-tricyclo [3.3.1.1^{3,7}] decane}-4-yl) disodium phenyl phosphate (commercial name: "AMPPD (Trademark)") are both available from Tropix Inc. With the use of CDP-Star or AMPPD for anti-GAD antibody measurement, luminescence intensity peaks can be significantly rapidly obtained and the luminescence intensity can be stably maintained for a long time period.

Furthermore, to enhance the chemiluminescence intensity of dioxycetane, an enhancer that provides a hydrophobic environment in which dioxycetane is sealed, such as a quaternary ammonium homopolymer and particularly poly(benzyltributyl) ammoniumchloride or a composition containing the same (SapphireII or EmeraldII (both are commercial names) available from Tropix Inc.) is preferably used.

### iii. Enzyme labeled antibody

Examples of an enzyme to be used in the present invention, which cleaves a specific group of dioxycetane to generate chemiluminescence, include alkaline or acid phosphatase, esterase, decarboxylase, phospholipase D, β-xylosidase, β-D-fucosidase, thioglucosidase, ATPase, ADPase, 5'nucleosidase, β-D-galactosidase, α-D-galactosidase, α-D-glucosidase, β-D-glucosidase, α-D-mannosidase, β-D-mannosidase, β-D-fructofuranosidase, β-D-glucoside uronase, and trypsin. In particular, phosphatase is preferable.

As an antibody labeled with such enzyme, a commercially available antibody may be used or prepared by a known method using an appropriate enzyme and an antibody. Such enzyme may be directly bound to an antibody or indirectly bound to the same via another substance. Examples of such another substance include avidin, streptavidin, and biotin.

### iv. Other reagents

As a sample for measuring the presence or the concentration of an anti-GAD antibody, a biological sample, particularly an appropriately diluted or undiluted serum, plasma, blood, or a lymph fluid is used. As a standard or a positive control anti-GAD antibody, which is used for measurement, any solution containing the anti-GAD antibody can be used. Particularly, when a sample is serum, a serum sample of a patient with insulin-dependent or non-insulin-dependent diabetes mellitus, which is positive for an anti-GAD antibody, is preferably used, As a negative control (negative for an anti-GAD antibody), a solution that is the same as that used for a positive control and contains no or substantially no anti-GAD antibody is used. An example of such solution is an anti-GAD antibody-negative human serum solution.

As a diluent for a sample, a phosphate buffer, a Tris hydrochloride buffer, a borate buffer, Good's buffer, a physiological saline containing one of them, or the like can be used. A diluent for a sample may be supplemented with serum, a preservative, and the like. Furthermore, the composition and the preparation method of a washing solution, a blocking solution, a buffer, or the like to be used for ELISA are known by persons skilled in the art. Such composition and preparation method suitable for implementation of the present invention can be arbitrarily selected and used.

### 3) Procedures for measuring anti-GAD antibodies

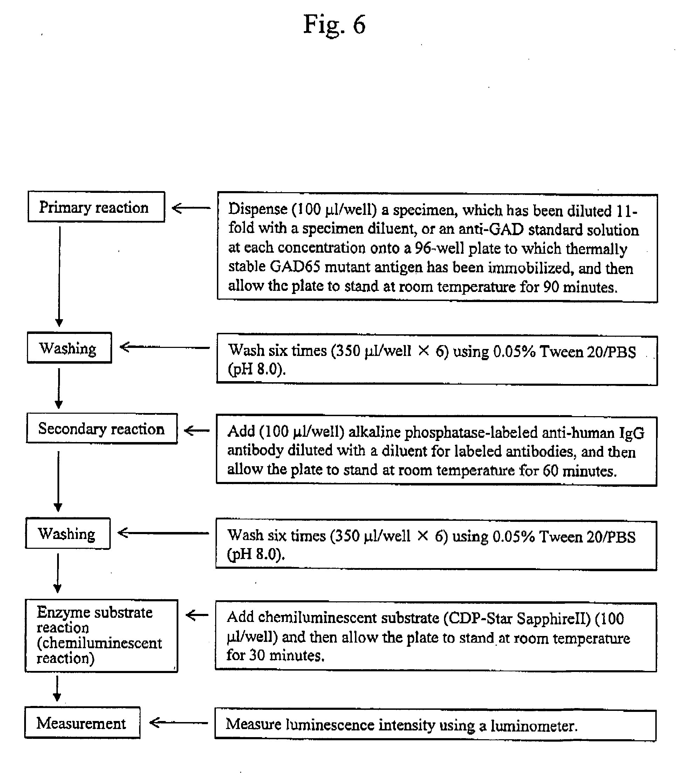

A method for measuring an anti-GAD antibody according to the present invention is described as follows. Specifically, a method as a typical example is explained, which involves measuring luminescence intensity according to the procedures described in Fig. 6 and calculating the concentration of an anti-GAD autoantibody based on the measured value.

First, a primary reaction is performed by binding an anti-GAD antibody in a sample to a thermally stable GAD65 mutant antigen immobilized on a carrier. An undiluted or appropriately diluted specimen (biological sample) is used as a sample. When quantitative measurement is performed, a standard solution (positive control sample) containing an anti-GAD antibody with a known concentration is tested simultaneously with a necessary negative control sample. The reaction is performed generally at a temperature between 20°C and 37°C. Room temperature is preferably employed in terms of convenience for procedures. Moreover, when quantitative measurement is performed using a microtiter plate, it is preferable to perform all the enzyme substrate reactions including a primary reaction; a secondary reaction (described later), and a luminous reaction at room temperature. Time for reaction is required to be as short as possible while stabilizing the reaction speed. It is required to appropriately determine optimum reaction time depending on the amount of an antigen to be immobilized and the amount of an antibody in a sample. In general, such time for reaction ranges from approximately 5 to 120 minutes. In the present invention, the time for reaction is preferably determined to be 60 to 120 minutes and particularly preferably determined to be approximately 90 minutes, for example. This is because the reaction speed of the primary reaction becomes sufficiently lowered at 90 minutes after the start of reaction and sufficient differences are confirmed in luminescence intensity depending on anti-GAD autoantibody concentrations when the relevant specimen contains an anti-GAD antibody at a concentration of approximately 100 units/ml.

Washing is performed using a buffer or the like for washing containing physiological saline buffered with phosphoric acid, boric acid, Tris hydrochloric acid, or the like and a surfactant. Washing is performed for separation and removal of components that have not bound to the carrier. The composition, amount, and washing time and/or washing frequency of a washing solution, and the like to achieve such purposes can be appropriately selected according to need. For example, washing is performed by adding an appropriate amount of Tris hydrochloride buffer or phosphate buffered saline (pH7.2 to 8.0) containing 0.05% to 0.5% Tween20 to a carrier once to six instances and then removing the same.

Next, a secondary reaction is performed. An enzyme labeled antibody is added to a carrier after washing and then bound to an anti-GAD antibody. Temperature for the reaction is as described above. Time for the reaction is generally preferably between 5 and 90 minutes and particularly preferably approximately 60 minutes in order to keep luminescence intensity at a level as low as possible when the concentration of the anti-GAD autoantibody is 0 unit/ml and to perform measurement within a short time.

Washing for the secondary reaction is performed in a manner similar to that after the primary reaction. Specifically, the carrier is washed to separate and remove excessive enzyme-labeled antibody that has remained unbound to the anti-GAD antibody.

An enzyme substrate reaction is performed by adding an appropriate amount of an enzyme substrate to a carrier after washing and then performing incubation for a predetermined time. Adequate time and temperature for the reaction can be appropriately determined, because such time and temperature can be varied depending on an enzyme, a substrate, and the type, amount, and composition of a buffer to be used herein. For example, an enzyme substrate reaction is performed in the present invention by adding chemiluminescent dioxycetane (produced by Tropix Inc., CDP-Star Sapphire II) with a concentration of 0.4 × 10⁻³ mol/L to microtiter wells at 100 µl/well and then causing the wells to stand at room temperature for 30 minutes.

The luminescence intensities of enzyme reaction products that are finally generated by an enzyme substrate reaction are measured using a luminometer or the like. The concentration of an anti-GAD antibody in a specimen to be analyzed can be measured using the fact that the amount of an antibody is directly proportional to luminescence intensity. The value (relative luminescence intensity: RLU) of a specimen to be analyzed, which is measured using a luminometer, can be converted into the concentration of an anti-GAD antibody based on a calibration curve graph showing the relationship between measured values of an anti-GAD antibody standard solution and the concentrations of an antibody in the relevant standard solution.

When particulates or the like to which a GAD65 mutant antigen has been immobilized is used as carriers, the concentration of the anti-GAD antibody can be measured by a method similar to the above method.

The present invention will be described in detail by examples as follows, but the scope of the present invention is not limited by these examples.

### Examples

### (Example 1)

### <Obtainment of GAD65 mutants>

(1) Plasmid pThioHisA-GAD65-Δ187 containing cDNA lacking the nucleotide sequence corresponding to amino acids 1 to 187 on the N-terminal side of GAD65 (hereinafter, the gene lacking such portion is referred to as GAD65-Δ187 gene (SEQ ID NO: 31)) was prepared as follows.
   A human GAD65 cDNA was prepared according to WO95/07992 and Bu et al., (Ding-Fang Bu, M. G. Erlander, B. C. Hitz, N. J. K. Tillakaratne, D. L. Kaufman, C. B. wagner-Mcpherson, G. A. Evans, A. J. Tobin (1992) and Two human glutamic decarboxylase, 65-kDa GAD and 67-kDa GAD, are each encoded by a single gene. Proc. Natl. Acad. U.S.A., 89, 2115-2119) and then subcloned into plasmid pPIC3.5K. PCR amplification was performed using the thus obtained pPIC3.5K-GAD65 as a template and GAD65-Δ187F (5'-TTGAATTCACCATGGTTGGATTAGCAGCAGACTGGC-3'; SEQ ID NO: 17) and 3' AOXR (5'-GGCAAATGGCATTCTGACATCCT-3'; SEQ ID NO: 18) primers. The thus obtained gene fragment was digested with *Eco*R I and *Not* I*,* incorporated into the *Eco*R I-*Not* I site of the above pThioHisA to transform *E. coli* JM109, and then cloning was performed.
(2) Next error-prone PCR was performed using the following materials and reaction system.
   Template plasmid DNA; pThioHisA-GAD65-Δ187
   Primers;
   Trx-F (5'-TTCCTCGACGCTAACCTG-3'; SEQ ID NO: 19)
   Trx-R (5'-TGTAAAACGACGGCCAGTGC-3'; SEQ ID NO: 20)
   Reaction system; {10 mM Tris·HCl (pH 8.3), 50 mM KCI, 1.5 mM MgCl₂, 0.01% BSA, 0.2 mM each dNTP mix, 10 ng/mL plasmid template, 0.32 µ M primers, 25 U/mL Taq DNA polymerase (TaKaRa), 50 µM MnCl₂}
   Reaction cycle; 94°C for 1 minute
   94°C for 30 seconds → 50°C for 30 seconds → 72°C for 4 minutes (25 cycles)
   The fragments generated by the above PCR were purified, digested with *Eco*R I and *Not* I, and then incorporated into a pThioHisA vector digested with the same enzymes, thereby constructing a plasmid DNA library.
   *E. coli* JM109 was transformed with the plasmid and then cultured in LB medium containing 100 µg/ml Amp (ampicillin) at 37°C for 15 hours.
(3) Next, a nitro cellulose membrane was placed on an LB medium plate coated with an IPTG solution as an expression inducer for *Escherichia coli* and containing 100 µg/ml Amp (ampicillin). The above transformant, the single colonies of pThioHisA-GAD65-Δ187/JM109, were inoculated on the membrane and then cultured at 37°C for 16 hours to cause colony formation. Furthermore, colonies on the nitro cellulose membrane were replicated on another nitro cellulose membrane.

The replica membrane on which the colonies had been formed was immersed in a lysis buffer {90% B-PER (PIERCE), 500 mM NaCl} and then lysis was performed at 25°C for 50 minutes. The membrane was washed twice with 50 mM Tris·HCl (pH 7.5), followed by blocking with a 10% skim milk solution. The nitro cellulose membrane was subjected to heat treatment at 45°C for 15 minutes.

Subsequently, the resultant was reacted with a primary antibody, 25-fold diluted positive serum of a patient (PSI), at 25°C for 1 hour. The resultant was washed 4 times with TBS-T and then reacted with a labeled secondary antibody (HRP-labeled anti-human IgG (each diluted 10, 000 folds) at 25°C for 1 hour. The resultant was washed 3 times with TBS-T and then subjected to detection using an ECL kit.

The number of the thus stained positive colonies was 128 out of approximately 72,000 colonies.

Subsequently, each colony replicated on the above nitro cellulose membrane and located at a position corresponding to each of the positive colonies was inoculated on similar medium described above, and then cultured under similar conditions.

Microbial bodies were harvested from each of the thus obtained culture product by centrifugation. The microbial bodies were disrupted by lysozyme treatment and ultrasonication. The resultant was purified using a Ni⁺⁺ chelate sepharose column that specifically adsorbs His-tag. Finally, six types of purified thermally stable GAD65-Δ187 mutants (F52, F68, F1, F86, F87, and F84) were obtained. The substitution in the amino acid sequence of each of these mutants and a change in the nucleotide sequence of each gene are as listed in Table 1 above.

The amino acid sequences of these mutants (F52, F68, F1, F86, F87, and F84) are represented by SEQ ID NOS: 6, 8, 10, 12, 14, and 16, respectively. The nucleotide sequences of the genes thereof are represented by SEQ ID NOS: 5, 7, 9, 11, 13, and 15, respectively.

### (Example 2)

The following example involves the preparation of a target protein by the PCR method using one of the mutants of the present invention, mutant F52, as a mutation primer. Mutant F52 retains particularly high thermostability among the other GAD65 mutants (GAD65-Δ187) lacking a region on the N-terminal side.

pThioHisA-GAD65-Δ187 obtained in Example 1 was used as a template.

Meanwhile, the following four types of primer were designed and then synthesized on commission. Underlined portions of primers F1 and R1 indicate an *Eco*R I recognition sequence and a *Not* I recognition sequence, respectively. The underlined portions of primers F2 and R2 indicate mutations to be introduced.
Primer F1; 5'-TTGAATTCACCATGGTTGGATTAGCAGACTGGC-3' (SEQ ID N0:21)
Primer F2; 5'-TCCTGGTTAGAGAAGCGGGATTGATGCAGAA-3' (SEQ ID NO: 22)
Primer R1; 5'-TAGCGGCCGCTTAGTGGTGGTGGTGGTGGTGAG-3' (SEQ ID NO: 23)
Primer R2; 5' -TTCTGCATCAATCCCGCTTCTCTAACCAGGA-3' (SEQ ID NO: 24)

The template gene was mixed with primers F1 and R2 and the template gene was mixed with primers F2 and R1. Each mixture was subjected to 25 cycles of PCR under the following conditions.

95°C for 30 seconds→55°C for 30 seconds→72°C for 2 minutes (25 cycles)

Two types of DNA fragment (700 base pairs and 518 base pairs) amplified by the aforementioned procedures were isolated. Subsequently, both fragments were mixed and then subjected to PCR under conditions similar to the above conditions. The DNA fragment of 1218 base pairs amplified by such procedures was isolated and digested with *Eco*R I and *Not* I. Meanwhile, *Eco*R I and *Not* I recognition sequences located at the multicloning site of plasmid pThioHisA for protein expression were digested with *Eco*R I and *Not* I. The DNA fragment that had thus been treated with the restriction enzymes was ligated using DNA ligase. The nucleotide sequence of the thus prepared mutant plasmid was confirmed. *E. coli* JM109 was transformed with the plasmid and then caused to undergo expression in a manner similar to that in Example 1. Therefore, the target mutant protein was obtained. It was revealed that the antigenicity of the mutant protein had retained high thermostability.

### (Example 3)

### <Improvement in solubility through deletion of regions on the of N-terminal sides of GAD65 mutants>

### (Preparation of each expression plasmid)

(1) hGAD65 cDNA was incorporated in frame into pThioHisA (Invitrogen) that had been digested with *EcoR* I and *Not* I (TaKaRa) and subjected to BAP treatment. Moreover, a gene encoding 6x polyhistidine tag was added to the 3'-terminus, so that pThioHisA-GAD65 was constructed. *E. coli* JM109 was transformed with the expression plasmid and then cloning was performed.
(2) PCR was performed using pPIC3.5K-GAD65 as a template and the following primers.
   G65-Δ95F;
   5'-TAGAATTCACCATGGTGCTGCGGGTGTGATGGAGAAAGGCCC-3' (SEQ ID NO: 25)
   3'AOXR;
   5'-GGCAAATGGCATTCTGACATCCT-3' (SEQ ID NO: 26)

Next, the thus obtained fragment was digested with *Eco*R I and *Not* I. The digested product was incorporated into the *Eco*R I*-Not* I site of the pThioHisA, thereby constructing expression plasmid pThioHisA-GAD65-Δ95. *E. coli* JM109 was transformed with the expression plasmid and then cloning was performed.

Moreover, pThioHisA-GAD65-Δ187 was constructed by the method described in Example 1(1). *E. coli* JM109 was transformed with the plasmid and then cloning was performed.

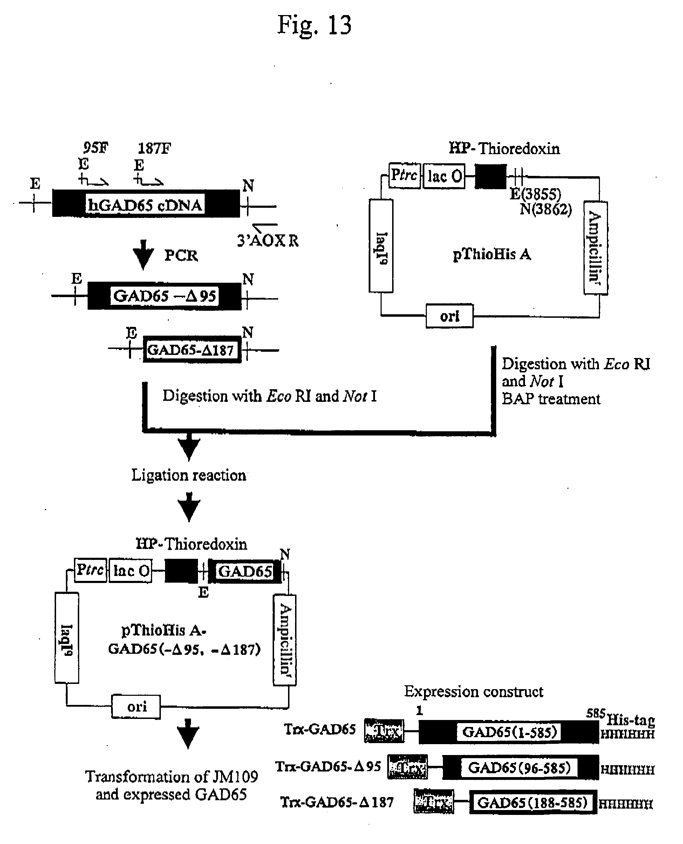

Fig. 13 shows the outline of the technique for constructing each of these expression plasmids.

### (Preparation of soluble (insoluble) fractions)

Next, microbial bodies into which the target gene had been introduced were pre-cultured overnight in 100 ml of LB (100 µg/ml and containing Amp.) medium at 37°C. 20 ml of a preculture solution was added for subculture to 400 ml of LB (100 mg/ml and containing Amp.) medium, followed by shake culture at 37°C. When OD reached OD600 = 0.5, IPTG (final concentration: 0.5 mM) was added for induction. At 2 hours after induction of expression, microbial bodies were harvested. The microbial bodies were suspended in 8 ml of a sonication buffer {50 mM Tris-HCl (pH 7.0), 100 mM NaCl, 1 mM EDTA, and 1 mM 2-mercaptoethanol} and then disrupted by ultrasonication under conditions of Output = 40%, duty cycle = 30%, and 10 minutes. Subsequently, 40 µl (final: 0.1%) of 20% Triton X-100 was added. The solution was allowed to stand on ice for 10 minutes and then centrifuged at 12000 g and 4°C for 10 minutes. Thus the supernatant was prepared as a soluble fraction. The precipitate was suspended again in 8 ml of sterilized water as long as possible, so that an insoluble fraction was obtained.

These fractions were subjected to 10% SDS-PAGE. Expression was confirmed by Western blotting using an anti-GAD monoclonal (MAB351) antibody (CHEMICON). Subsequently films were measured using a densitometer. Solubilization% and insolubilization% were calculated based on the concentration of each band (relative value).
Table 2 shows the results.

**Table 2**

| | GAD65 | GAD65-Δ95 | GAD65-Δ187 |
|---|---|---|---|
| Solubilization% | 12 | 27 | 16 |
| Insolubilization% | 88 | 73 | 54 |

As is clear from the experimental results, deletion of a region on the N-terminal side of GAD65 resulted in increased solubilization% and deletion of 197 amino acid residues from the N terminus enabled solubilization of approximately 50% of the proteins.

### (Example 4)

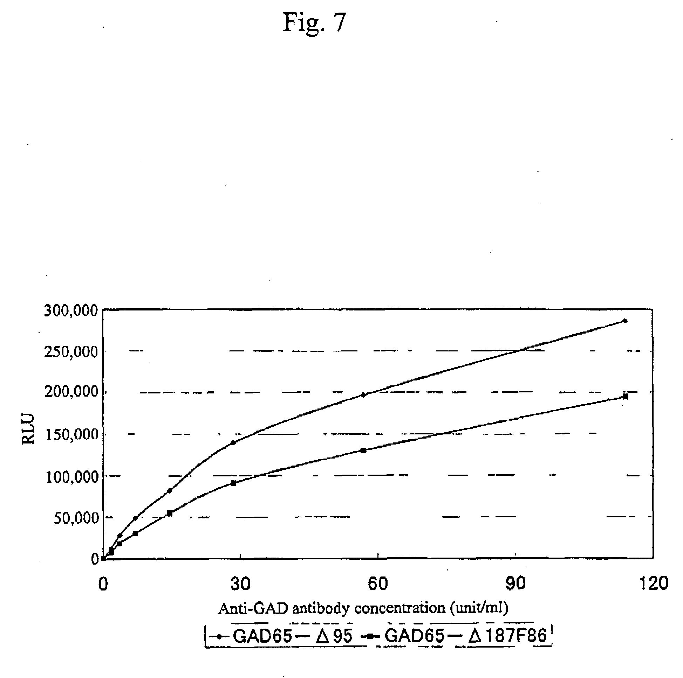

(1) F86 was selected from thermally stable GAD65-Δ187 mutants obtained in Example 1 and then subjected to measurement relating to the amount of a GAD antigen immobilized (undertaken by a chemiluminescence ELISA method) and reactivity with the positive serum of IDDM patients.
The mutant F86 showed the highest level of binding with the GAD-positive serum of a patient at an immobilization concentration between 0.1 and 1.0 µg/well.
In addition, ideally, a full-length GAD65 antigen is used as a control. However, the full-length GAD65 has high hydrophobicity, so that retention of its antigenicity is difficult. Hence, the above GAD65-Δ95 antigen was used instead.
The GAD65-Δ95 antigen (SEQ ID NO: 4) was obtained as follows. A c-DNA fragment of the GAD65-Δ95 gene (SEQ ID NO: 3) was incorporated into expression vector pHIL-D2. Then the vector was introduced into a host *Pichia pastoris* GS115 strain. A GAD65-Δ95-expressing strain was screened for from the thus obtained colonies. Therefore, the target GAD65-Δ95-expressing strain was obtained.
Methanol was added to GAD65-Δ95-expressing Pichia strain, so as to induce the generation of the target protein (GAD antigen). Cultured microbial bodies were then harvested. The obtained microbial bodies were disrupted using glass beads. The supernatant portion (Pichia microbial body extract) was adsorbed to a Ni⁺⁺ cellulose column. The target GAD antigen was eluted with imidazole. GAD65-Δ95 purified with the Ni⁺⁺ cellulose column was further purified with an anti-GAD monoclonal antibody column. The thus obtained GAD65-Δ95 antigen was immobilized at a concentration between 0.5 mg/well and 2.0 mg/well as measured by the ELISA method. The GAD65-Δ95 antigen showed the highest level of binding with the GAD-positive serum of patients.
(2) Furthermore, a dilution series was prepared via serial dilution (diluted 1/2, 1/4,...) using 114 units/ml GAD-positive serum specimens of patients (based on the patient's serum antibody titer as defined by International Diabetes Federation; IDF). The GAD65-Δ95 antigen and the GAD65-Δ187 mutant strain F86 antigen were separately immobilized and then the reactivity to human anti-GAD anti-serum was examined by the chemiluminescence ELISA method. Table 3 shows the results. Moreover, based on the results, a dilution curve of an anti-GAD-antibody-positive serum specimen of a patient was generated for a case of using the GAD65-Δ95 antigen and a case of using the GAD65-Δ187 mutant strain F86 antigen (Fig. 7).
Differences were observed in luminescence intensity because the immobilization conditions for both the antigens had not been adjusted equivalently and a Ni⁺⁺ cellulose column alone had been used for the purification of the mutant strain. However, similar tendencies were observed in terms of correlation between the serum dilution series and luminescence intensity. It was thus confirmed that the mutant strain had antigenicity equivalent to that of GAD65-Δ95.

**Table 3**

| Unit/ml | GAD65-Δ95 | GAD65-Δ187 F86 |
|---|---|---|
| 0 | 125 | 75 |
| 1.8 | 12,940 | 8,260 |
| 3.6 | 28,035 | 19,010 |
| 7.1 | 48,950 | 30,410 |
| 14.3 | 81,635 | 54,425 |
| 28.5 | 139,400 | 90,885 |
| 57 | 196,560 | 130,235 |
| 114 | 285,930 | 194,745 |

| | | |
|---|---|---|
| Unit/ml: anti-GAD antibody titer Unit: RLU | | |

(3) The reactivity of the GAD65-Δ187 mutant strain F86 antigen and the reactivity of the GAD65-Δ95 antigen to human anti-GAD anti-serum were compared and examined using serum specimens (GAD-negative) from twenty-three healthy subjects and clinical specimens (GAD-positive) from six GAD patients. Table 4 shows the results. Next, the sensitivity and specificity between them were calculated using anti-GAD antibody titer of 5.0 unit/ml as the cut-off value for healthy subjects. As shown in Table 5 below, the sensitivity of the GAD antigen of GAD65-Δ187 mutant strain F86 was 100% and the specificity of the same was 91% compared with the control GAD65-Δ95. Both sensitivity and specificity of F86 were 90% or more, compared with the control GAD65-Δ95. It was confirmed that the antigenicity of the mutant strain had been equivalent to that of GAD65-Δ95.

**Table 4**

| Measurement of anti-GAD antibody titer of clinical specimens | | | | | |
|---|---|---|---|---|---|
| | | GAD65-Δ95 | | GAD65-Δ187 F86 | |
| No. | Specimen | RLU | Unit/ml | RLU | Unit/ml |
| 1 | Negative | 14,100 | 1.9 | 9,520 | 2.5 |
| 2 | Negative | 20,940 | 2.8 | 11,490 | 3 |
| 3 | Negative | 8,360 | 1.2 | 6,710 | 1.8 |
| 4 | Negative | 26,740 | 3.6 | 10,980 | 2.9 |
| 5 | Negative | 20,590 | 2.7 | 10,980 | 2.9 |
| 6 | Negative | 10,000 | 1.4 | 9,120 | 2.4 |
| 7 | Negative | 14,260 | 2.7 | 17,970 | 2.3 |
| 8 | Negative | 19,680 | 2.6 | 8,700 | 2.3 |
| 9 | Negative | 18,570 | 2.5 | 11,660 | 3.2 |
| 10 | Negative | 22,550 | 4.4 | 21,480 | 5.5 |
| 11 | Negative | 41,690 | 5.9 | 19,480 | 5 |
| 12 | Negative | 35,660 | 5 | 18,630 | 4.9 |
| 13 | Negative | 5,490 | 1 | 5,520 | 1.5 |
| 14 | Negative | 27,170 | 3.7 | 13,520 | 3.5 |
| 15 | Negative | 19,250 | 2.6 | 9,410 | 2.5 |
| 16 | Negative | 35,240 | 4.9 | 12,150 | 3.1 |
| 17 | Negative | 18,940 | 2.6 | 7,670 | 2.1 |
| 18 | Negative | 19,500 | 2.1 | 12,680 | 3.3 |
| 19 | Negative | 15,960 | 2.2 | 11,290 | 3 |
| 20 | Negative | 18,860 | 2.5 | 9,890 | 2.6 |
| 21 | Negative | 20,730 | 2.8 | 21,490 | 7.1 |
| 22 | Negative | 31,350 | 4.3 | 16,930 | 4.3 |
| 23 | Negative | 36,140 | 4.9 | 17,460 | 4.5 |
| 24 | Positive | 209,050 | 60.5 | 37,060 | 10.1 |
| 25 | Positive | 54,640 | 8.2 | 29,070 | 7.7 |
| 26 | Positive | 375,290 | Over | 329,550 | Over |
| 27 | Positive | 61,590 | 9.4 | 34,660 | 9.3 |
| 28 | Positive | 165,510 | 39.6 | 109,890 | 41.4 |
| 29 | Positive | 188,560 | 49.9 | 149,030 | 67 |

| | | | | | |
|---|---|---|---|---|---|
| RLU: Relative luminescence intensity Unit/ml: GAD antibody titer | | | | | |

**Table 5**

| | GAD65-Δ95 | | | Total |
|---|---|---|---|---|
| | | + | - | |
| GAD65-Δ187 | + | 7 | 2 | 9 |
| F86 | - | 0 | 20 | 20 |
| | Total | 7 | 22 | 29 |

| | | | | |
|---|---|---|---|---|
| Sensitivity = 100% (7/7) Specificity = 90.9% (20/22) | | | | |

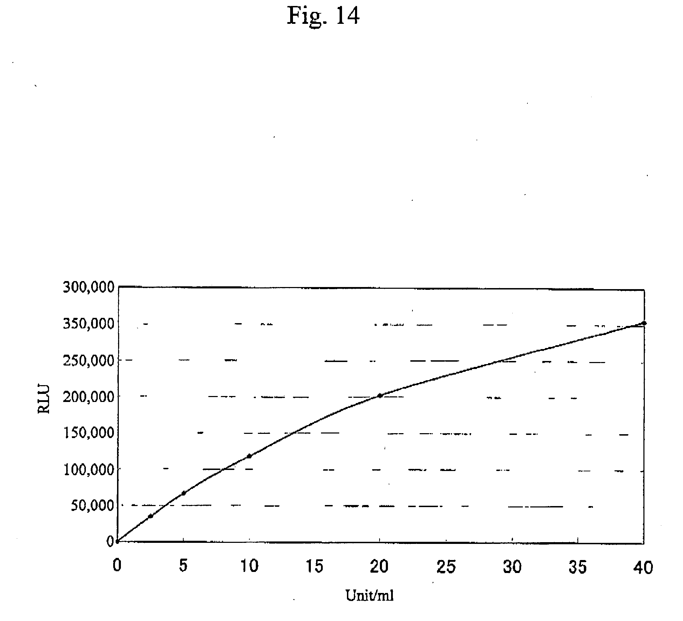

(4) The reactivity of the positive serum of a patient to the GAD65 antigen obtained from the GAD65-Δ187 mutant F86 strain is shown in (2) above. The concentration of the antigen immobilized was examined again based on the results. The highest level of binding was obtained with an immobilization amount of 0.2 µg/well. For measurement of anti-GAD antibody titer, it is necessary to emphasize the stability of measurement values within a low value range near the cut-off value. Concentrations were determined using a 5-point dilution series starting from 40 units/ml (40, 20, 10, 5, and 2.5 units/ml), and then measurement was performed. Table 6 shows the results. Furthermore, based on the results in Table 6, an anti-GAD antibody standard calibration curve was generated (Fig. 14). According to this curve, sufficient differences in luminescence intensity were obtained for different concentrations from 2.5 units/ml to 40 units/ml. With the use of a calibration curve, an antibody titer of an anti-GAD antibody in the serum of a patient can be immediately found based on measured luminescence intensity.

**Table 6**

| Unit/ml | RLU |
|---|---|
| 0 | 120 |
| 2.5 | 34,680 |
| 5 | 66,390 |
| 10 | 118,190 |
| 20 | 202, 150 |
| 40 | 304, 920 |

### (Example 5)

### <Antigenicity of GAD65 mutants (GAD65-Δ187) lacking regions on the N-terminal sides>

Each purified GAD65 mutant (GAD65-Δ187) lacking the relevant region on the N-terminal side (obtained in Example 1) was diluted with an immobilization buffer (20 mM Tris-HCl (pH 7.6), 13.7 mM NaCl, 0.1% Triton X-100, and 0.05% CHAPS) at a concentration of 0.25 µg/100 µl. The diluted solution was dispensed at 100 µl/well onto a 96-well plate, followed by overnight immobilization at 4°C. Subsequently, a blocking solution (50 mM Tris-HCl (pH 7.5), 150 mM NaCl, 0.1% Triton X-100, and 3% BSA) was dispensed at 300 µl/well onto the 96-well plate, followed by 2 hours of blocking at 4°C.

The serum of a PSI patient (70 unit/ml) (based on the antibody titer of a patient's serum as defined by the International Diabetes Federation; IDF) was dispensed as a primary antibody at 100 µl/well onto the 96-well plate, followed by 1.5 hours of reaction at 25°C. Subsequently, the wells were washed three times using TBS-T. ALP-labeled anti-human IgG antibody (diluted 5000-fold) was dispensed as a secondary antibody at 100 µl/well onto the 96-well plate, followed by 1 hour of reaction at 25°C. Next, the wells were washed 5 times using TBS-T. CDP-star (Tropix) was then dispensed as a substrate at 100 µl/well onto the 96-well plate, followed by 30 minutes of reaction at 25°C. Luminescence intensity was then measured using a Plate Reader (DIA-IATRON Luminous CT-9000D).

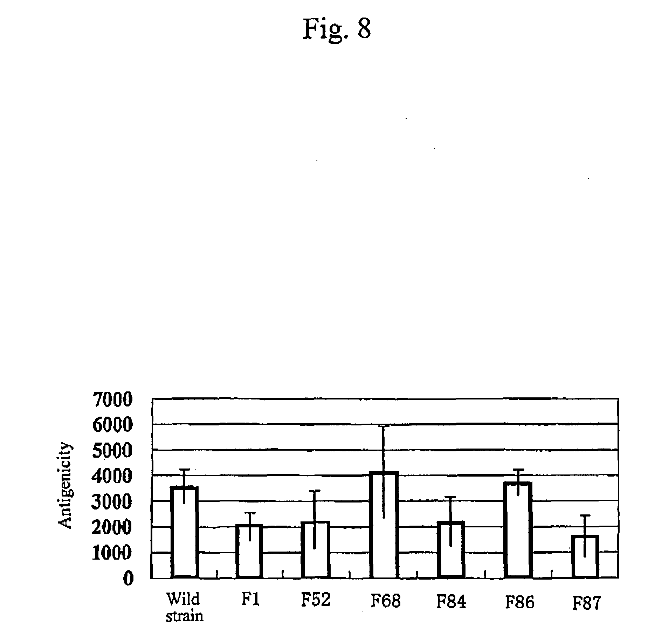

Fig. 8 shows the results. Fig. 8 also shows the antigenicity of control GAD65-Δ187 (wild type; SEQ ID NO: 32) having no mutations, for which similar procedures were performed.

Increased antigenicity was observed for mutants F68 and F86, compared with that of GAD65-Δ187 (wild type; SEQ ID NO: 32) having no mutations. On the other hand, relatively decreased antigenicity was observed for mutants F1, F52, F84, and F87, but the amount of such antigenicity relative to that of the wild type was approximately one-half thereof at maximum. It was revealed that the mutants had sufficient antigenicity.

### (Example 6)

### <Thermostability of GAD65 mutants (GAD65-Δ187) lacking regions on the N-terminal sides>

Each of the purified GAD65 mutants (GAD65-Δ187) lacking the relevant regions on the N-terminal sides obtained in Example 1 was diluted with an immobilization buffer (20 mM Tris-HCl (pH 7.6), 13.7 mM NaCl, 0.1% Triton X-100, and 0.05% CHAPS) at a concentration of 0.25 µg/100 µl, followed by incubation using warm water at 45°C for 0 minutes, 10 minutes, and 30 minutes. Subsequently, the solution was dispensed at 100 µl/well onto a 96-well plate, followed by overnight immobilization at 4°C. Subsequently, a blocking solution (50 mM Tris-HCl (pH 7.5), 150 mM NaCl, 0.1% Triton X-100, and 3% BSA) was dispensed at 300 µl/well onto the 96-well plate, followed by blocking at 4°C for 2 hours. Procedures similar to those in Example 3 were then performed and then luminescence intensity was measured.

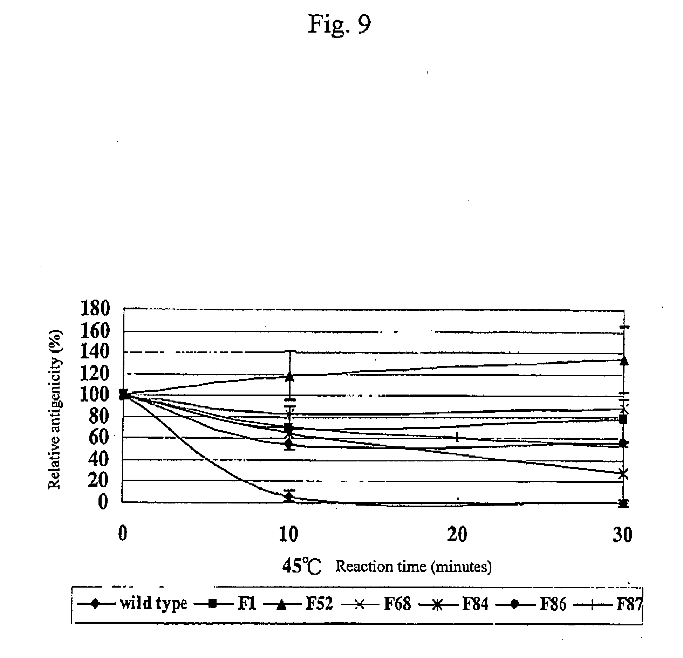

Fig. 9 shows fluctuations in antigenicity, resulting from heat treatment of the GAD65 mutants at 45°C. In addition, Fig. 9 also shows fluctuations in antigenicity of control GAD65-Δ187 (wild type; SEQ ID NO: 32) having no mutations, for which similar procedures were performed.

As shown in Fig. 9, the half-life of the antigenicity of GAD65-Δ187 (wild type) having no mutations was approximately 1 minute (data not shown), and GAD65-Δ187 lost its antigenicity almost completely after 10 minutes of heat treatment. In contrast, the half-lives of all the mutants were 10 minutes or more. Even after 30 minutes of heat treatment, mutants F1 and F68 retained approximately 80% antigenicity and mutants F86 and F87 retained approximately 50% antigenicity. Specifically, the mutants were thermally stable at levels 30 times or more than the wild type. It is striking that the antigenicity of mutant F52 became higher with the progression of heat treatment, such that after 30 minutes of heat treatment, mutant F52 exerted antigenicity 1.3 times greater than that before heat treatment. As described above, it is clear that these mutants acquired high thermostability.

### (Example 7)

### <Fluctuations in antigenicity of a GAD65 mutant (GAD65-Δ187) lacking a region on the N-terminal side with lengthy heat treatment at 45°C>

F52 exerted particularly high thermostability among the purified GAD65 mutants (GAD65-Δ187) lacking the regions on the N-terminal side obtained in Example 1. F52 was diluted with an immobilization buffer (20 mM Tris-HCl (pH 7.6), 13.7 mM NaCl, 0.1% Triton X-100, and 0,05% CHAPS) at a concentration of 0.25 µg/100 µl, followed by incubation using warm water at 45°C for 1 hour, 3 hours, and 6 hours. Subsequently, the solution was dispensed at 100 µl/well onto a 96-well plate, followed by overnight immobilization at 4°C. Subsequently, a blocking solution (50 mM Tris-HCl (pH 7.5), 150 mM NaCl, 0.1% Triton X-100, and 3% BSA) was dispensed at 300 µl/well onto the 96-well plate, followed by 2 hours of blocking at 4°C. Procedures similar to those in Example 3 were performed and then luminescence intensity was measured.

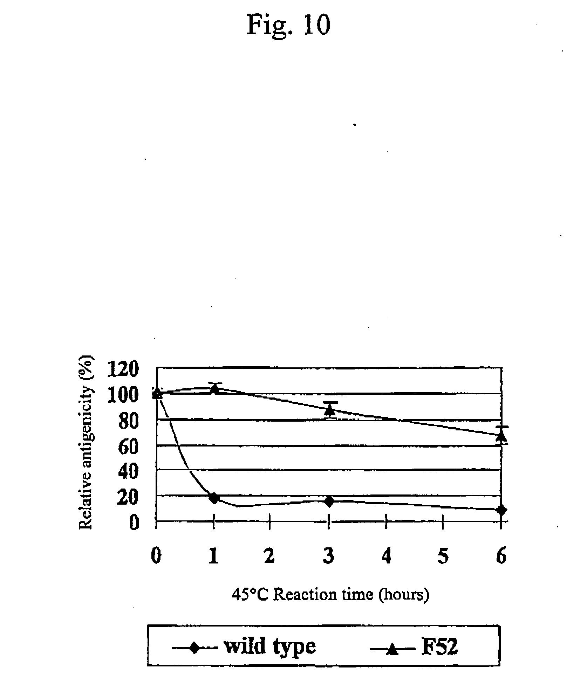

Fig. 10 shows fluctuations in antigenicity of GAD65-Δ187 (F52) as a result of lengthy heat treatment at 45°C. In addition, Fig. 10 also shows fluctuations in antigenicity of control GAD65-Δ187 (wild type; SEQ ID NO: 32) having no mutations, for which similar procedures were performed.

The antigenicity of GAD65-Δ187 (wild type) having no mutations decreased to around 20% antigenicity after 1 hour of heat treatment. However, F52 retained 90% antigenicity after 3 hours of heat treatment and retained 70% antigenicity even after 6 hours of heat treatment. Thus, it was revealed that F52 had preserved surprisingly high thermostability. Such high thermostability was far beyond the level originally predicted.

### (Example 8)

### <Fluctuations in antigenicity of a GAD65 mutant (GAD65-Δ187) lacking a region on the N-terminal-side with high-temperature (60°C) heat treatment>

F52 exerted particularly high thermostability after heat treatment at 45°C among the purified GAD65 mutants (GAD65-Δ187) lacking the regions on N-terminal sides obtained in Example 1. F52 was diluted with an immobilization buffer (20 mM Tris-HCl (pH 7.6), 13.7 mM NaCl, 0.1% Triton X-100, and 0.05% CHAPS) at a concentration of 0.25 µg/100 µl, followed by heat treatment using warm water at 60°C for varied time lengths up to 30 minutes. Subsequently, the solution was dispensed at 100 µl/well onto a 96-well plate, followed by overnight immobilization at 4°C. Subsequently, a blocking solution (50 mM Tris-HCl (pH 7.5), 150 mM NaCl, 0.1% Triton X-100, and 3% BSA) was dispensed at 300 µl/well onto the 96-well plate, followed by 2 hours of blocking at 4°C. The procedures similar to those in Example 3 were performed and then luminescence intensity was measured.

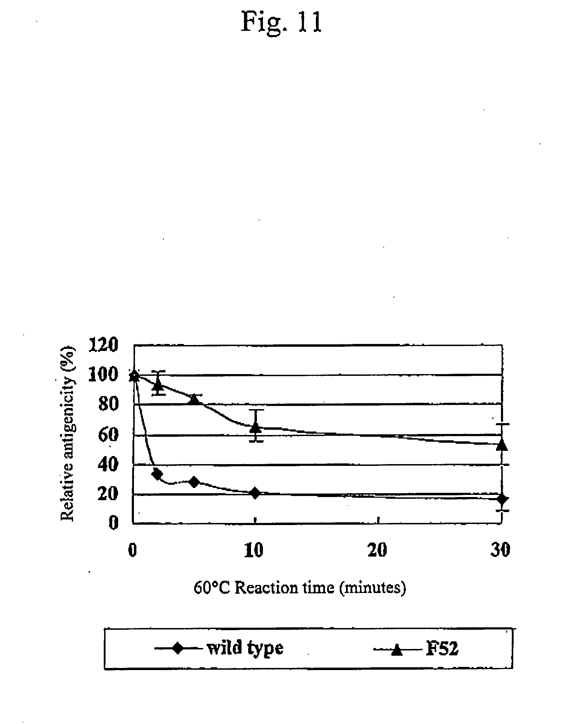

Fig. 11 shows the results. In addition, Fig. 11 also shows fluctuations in antigenicity of control GAD65-Δ187 (wild type; SEQ ID NO: 32) having no mutations, for which similar procedures were performed.

As shown in Fig. 11, the antigenicity of GAD65-Δ187 (wild type) having no mutations decreased rapidly. However, F52 retained approximately 50% antigenicity even after 30 minutes of heat treatment. It was revealed that F52 had preserved the thermostability at a surprisingly high temperature. The thermostability exerted at such high temperature was far beyond the level originally predicted.

### (Example 9)

### <Antigenicity of a GAD65 mutant (GAD65-Δ187) lacking a region on the N-terminal side with heat treatment in the presence of 10% methanol>

F52 exerted particularly higher thermostability after heat treatment at 60°C than other purified GAD65 mutants (GAD65-Δ187) lacking the regions on the N-terminal sides obtained in Example 3. F52 was diluted with an immobilization buffer containing 10% methanol (20 mM Tris-TCl (pH 7.6), 13.7 mM NaCl, 0.1% Triton X-100, and 0.05% CHAPS) at a concentration of 0.25 µg/100 µl, followed by heat treatment using warm water at 45°C for varied time lengths up to 30 minutes. Subsequently, the solution was dispensed at 100 µl/well onto a 96-well plate, followed by overnight immobilization at 4°C. Subsequently, a blocking solution (50 mM Tris-HCl (pH 7.5), 1.50 mM NaCl, 0.1% Triton X-100, and 3% BSA) was dispensed at 300 µl/well onto the 96-well plate, followed by 2 hours of blocking at 4°C. Procedures similar to those in Example 3 were performed and then luminescence intensity was measured.

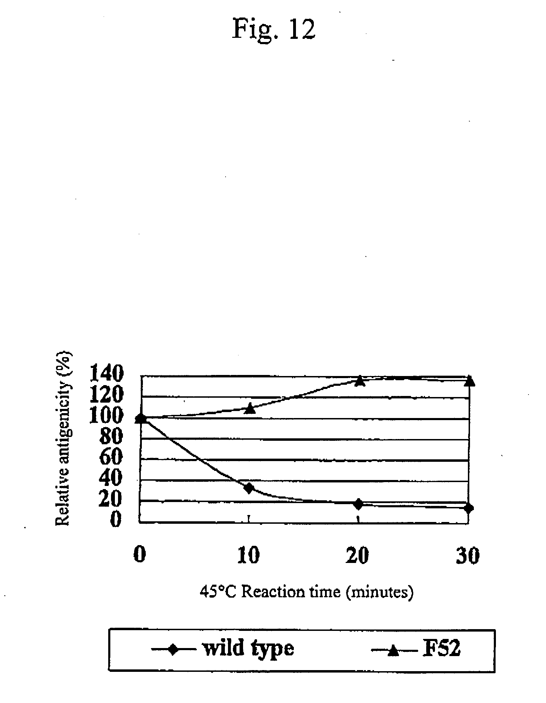

Fig. 12 shows fluctuations in antigenicity due to heat treatment conducted for GAD65 mutant F52.

In addition, Fig. 12 also shows fluctuations in antigenicity of control GAD65-Δ187 (wild type; SEQ ID NO: 32) having no mutations, for which similar procedures were performed.

Antigenicity of GAD65-Δ187 (wild type) having no mutations rapidly decreased. However, GAD65 mutant F52 retained the original antigenicity level even after 30 minutes of heat treatment in the presence of 10% methanol. It was revealed that GAD65 mutant F52 had retained thermostability. Retention of antigenicity even in the presence of such an organic solvent is extremely advantageous for practical use.

### Industrial Applicability

The present invention makes it possible to provide thermally stable GAD65 mutants that retain antigenicity to pancreatic islet-β-cell-specific autoantibodies in the serum of a patient. The present invention also makes it possible to provide solubilized GAD65 mutants retaining antigenicity and thermostability.

Pancreatic islet-β-cell-specific autoantibodies in the serum of a patient can be detected and quantified with better accuracy and sensitivity through the use of such GAD65 mutants. Therefore, these GAD65 mutants are extremely useful as diagnostic agents for insulin-dependent diabetes mellitus. In addition, the solubilized GAD mutants can be simply separated and purified, so that such mutants with high purities can be obtained.

All publications, patents, and patent applications cited herein are incorporated herein by reference in their entirety.

## Claims

1. A glutamic acid decarboxylase 65 mutant, which has a mutation in the region between amino acid residues 310 and 470 in the amino acid sequence represented by SEQ ID NO: 2 in Sequence Listing and antigenicity to a pancreatic islet-β-cell-specific autoantibody and retains 50% or more of the relative antigenicity when heated at 45°C for 10 minutes.

2. A glutamic acid decarboxylase 65 mutant, which has any one mutation of the following amino acid mutations (a) to (f) in the amino acid sequence represented by SEQ ID NO: 2:
(a) E412G;
(b) S313F;
(c) M361I and/or F467S;
(d) K355R;
(e) D345V; and
(f) I353T.

3. A glutamic acid decarboxylase 65 mutant, which lacks a region on the N terminal side of the glutamic acid decarboxylase 65 mutant according to claim 2 above and is solubilized.

4. The glutamic acid decarboxylase 65 mutant according to claim 3, which lacks amino acid residues 1 to 187.

5. A glutamic acid decarboxylase 65 mutant, in which thioredoxin is added to the N terminus of the glutamic acid decarboxylase 65 mutant according to any one of (1) to (3) above.

6. A reagent for detection or quantification of a pancreatic islet-β-cell-specific autoantibody, which contains the glutamic acid decarboxylase 65 mutant according to any one of claims 1 to 5.

7. A diagnostic agent for insulin-dependent diabetes mellitus, which contains the glutamic acid decarboxylase 65 mutant according to any one of claims 1 to 5.

8. A DNA, which has a nucleotide sequence encoding the glutamic acid decarboxylase 65 mutant according to any one of claims 1 to 5,

9. A DNA, which has any one of the following mutations (a) to (f) in the nucleotide sequence represented by SEQ ID NO: 1:
(a) a mutation wherein GAG between nucleotides 1234 and 1236 in the nucleotide sequence represented by SEQ ID NO: 1 is substituted with GGA, GGC, GGG, or GGT;
(b) a mutation wherein TCT between nucleotides 937 and 939 in the aforementioned nucleotide sequence is substituted with TTC or TTT;
(c) a mutation wherein ATG between nucleotides 1081 and 1083 in the aforementioned nucleotide sequence is substituted with ATA, ATC, or ATT, and/or TTT between nucleotides 1399 and 1401 in the aforementioned nucleotide sequence is substituted with AGC, AGT, TCA, TCC, TCG, or TCT;
(d) a mutation wherein AAA between nucleotides 1063 and 1065 in the aforementioned nucleotide sequence is substituted with AGA, AGG, CGA, CGC, CGG, or CGT;
(e) a mutation wherein GAC between nucleotides 1033 and 1035 in the aforementioned nucleotide sequence is substituted with GTA, GTC, GTG, or GTT; and
(f) a mutation wherein ATT between nucleotides 1057 and 1059 in the aforementioned nucleotide sequence is substituted with ACA, ACC, ACG, or ACT.

10. The DNA according to claim 9, which lacks a nucleotide sequence ranging from nucleotides 1 to 561 in the nucleotide sequence represented by SEQ ID NO: 1.

11. The DNA according to claim 9 or 10, which has the nucleotide sequence represented by SEQ ID NO: 27 on the 5' terminal side.

12. An antisense DNA or an antisense RNA, which forms an antisense strand in relation to the DNA according to any one of claims 8 to 11,

13. A recombinant vector, into which the DNA according to any one of claims 8 to 11 is inserted.

14. A transformant, which is transformed with the recombinant vector according to claim 13.

15. A method for producing a glutamic acid decarboxylase 65 mutant, which comprises culturing the transformant according to claim 14 in medium and then harvesting the glutamic acid decarboxylase 65 mutant according to any one of claims 1 to 5 from the culture product.
